Europäisches Patentamt
European Patent Office
Office européen des brevets

(19)

(11) Veröffentlichungsnummer: **0 254 682**
**A1**

(12)

# EUROPÄISCHE PATENTANMELDUNG

(21) Anmeldenummer: **87810398.5**

(22) Anmeldetag: **16.07.87**

(51) Int. Cl.[4]: **C 07 D 211/90**
C 07 D 401/04,
C 07 D 405/04,
C 07 D 413/04,
C 07 D 401/12,
C 07 D 401/14,
C 07 D 405/14,
C 07 D 413/14, A 61 K 31/44
// C07C125/065, C07C79/343,
C07C93/06

(30) Priorität: **22.07.86 CH 2918/86**

(43) Veröffentlichungstag der Anmeldung:
**27.01.88 Patentblatt 88/04**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Ostermayer, Franz, Dr.**
**Am Hang 5**
**CH-4125 Riehen (CH)**

**Zimmermann, Markus, Dr.**
**Steinbrecheweg 8**
**CH-4125 Riehen (CH)**

(54) **Neue Phenoxyaliphatylphenylenoxyalkylester und -amide.**

(57) 1,4-Dihydropyridin-3-carbonsäurephenoxyaliphatylphenylenoxyalkylester der Formel

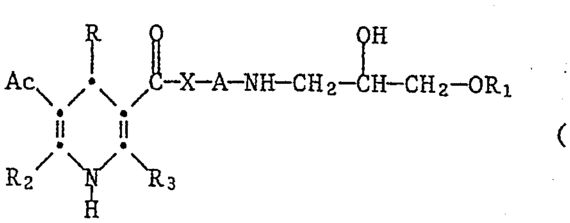

(I)

worin Ac eine Acylgruppe bedeutet, X Oxy oder gegebenenfalls substituiertes Imino bedeutet, A einen durch eine Gruppe $-X_1-Ph-X_2-$, worin mindestens einer der Reste $X_1$ und $X_2$ Oxy und ein davon verschiedener Rest $X_1$ oder $X_2$ eine direkte Bindung darstellt und Ph einen gegebenenfalls substituierten Phenylenrest bedeutet, unterbrochenen zweiwertigen aliphatischen Kohlenwasserstoffrest darstellt, R einen gegebenenfalls substituierten Phenyl-, Pyridyl-bzw. 1-Oxidopyridyl-, (1,3-Dioxa)indanyl- oder Benzofurazanylrest bedeutet, $R_1$ einen ggf. substituierten Phenyl- oder Indolylrest bedeutet, $R_2$ einen aliphatischen Kohlenwasserstoffrest bedeutet und $R_3$ einen aliphatischen Rest, Cyano oder Amino bedeutet, und ihre Säureadditionssalze können als Arzneimittelwirkstoffe verwendet werden und werden in an sich bekannter Weise hergestellt.

EP 0 254 682 A1

**Beschreibung**

Neue Phenoxyaliphatylphenylenoxyalkylester und -amide

Die Erfindung betrifft neue 1,4-Dihydropyridin-3-carbonsäurephenoxyaliphatylphenylenoxyalkylester der Formel

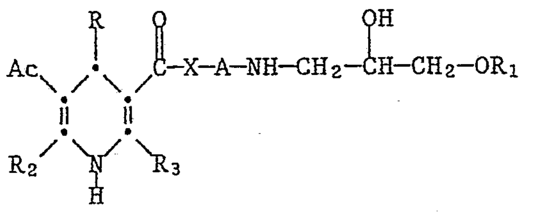

(I),

worin Ac eine Acylgruppe bedeutet, X Oxy oder gegebenenfalls substituiertes Imino bedeutet, A einen durch eine Gruppe $-X_1$-Ph-$X_2$-, worin mindestens einer der Reste $X_1$ und $X_2$ Oxy und ein davon verschiedener Rest $X_1$ oder $X_2$ eine direkte Bindung darstellt und Ph einen gegebenenfalls substituierten Phenylenrest bedeutet, unterbrochenen zweiwertigen aliphatischen Kohlenwasserstoffrest darstellt, R einen gegebenenfalls substituierten Phenyl-, Pyridyl-bzw. 1-Oxidopyridyl-, (1,3-Dioxa)indanyl- oder Benzofurazanylrest bedeutet, $R_1$ einen ggf. substituierten Phenyl- oder Indolylrest bedeutet, $R_2$ einen aliphatischen Kohlenwasserstoffrest bedeutet und $R_3$ einen aliphatischen Rest, Cyano oder Amino bedeutet, und ihre Säureadditionssalze.

Acylgruppen Ac leiten sich beispielsweise von aliphatischen Carbon-oder Sulfonsäuren oder von Halbestern der Kohlensäure ab und bedeuten z.B. Niederalkoxycarbonyl, Niederalkansulfonyl oder Niederalkanoyl.

Substituiertes Imino ist beispielsweise aliphatisch substituiert und bedeutet z.B. Niederalkylimino.

Phenylen ist 1,2-, 1,3- und insbesondere 1,4-Phenylen.

Als Substituenten aromatischer Reste Ph, R und $R_1$ kommen beispielsweise für Ph Niederalkyl, Niederalkoxy, Halogen, Trifluormethyl und/oder Nitro, für R Niederalkyl, Niederalkoxy, Halogenniederalkoxy, Niederalkenyloxy, Halogen, Trifluormethyl, Cyano, gegebenenfalls durch Niederalkyl, Niederalkoxy, Halogen, Trifluormethyl und/oder Nitro substituierte Benzylthio- bzw. Benzyloxyreste und/oder Nitro und für $R_1$ Niederalkyl, Niederalkoxyniederalkyl, Niederalkoxyniederalkoxyniederalkyl, Cycloalkylniederalkoxyniederalkyl, Carbamylniederalkyl, Niederalkanoylaminoniederalkyl, Niederalkoxy, Niederalkenyloxy, Niederalkinyloxy, Niederalkoxyniederalkoxy, Carbamylniederalkoxy, Niederalkanoylaminoniederalkoxy, Halogen, Niederalkanoyl und/oder Cyano in Frage.

Zweiwertige aliphatische Kohlenwasserstoffreste als Bestandteil von A sind beispielsweise Alkylenreste, beispielsweise $C_2$-$C_{14}$-Alkylen, insbesondere Niederalkylen.

Aliphatische Kohlenwasserstoffreste $R_2$ sind beispielsweise Niederalkylreste.

Aliphatische Reste $R_3$ sind beispielsweise gegebenenfalls elektronegativ substituierte, wie hydroxylierte, Niederalkylreste, insbesondere Niederalkyl und Hydroxyniederalkyl.

Reste A sind beispielsweise Niederalkylenoxyphenylenoxyniederalkylen- oder Niederalkylen- bzw. Niederalkylidenphenylenoxyniederalkylenreste. Reste -X-A- sind dementsprechend beispielsweise solche der Formeln -X-$alk_1$-O-Ph-$alk_2$- oder -X-$alk_1$-O-Ph-O-$alk_3$-, worin $alk_1$ und $alk_3$ gleiche oder verschiedene Niederalkylenreste bedeuten, $alk_2$ Niederalkylen oder Niederalkyliden darstellt und X und Ph vorstehende Bedeutungen haben.

Phenyl-, Pyridyl- bzw. 1-Oxidopyridylreste R, Phenyl- bzw. Indolylreste $R_1$ sowie Phenylenreste Ph als Bestandteil der Gruppe A können bis und mit 3, insbesondere 1 oder 2, der genannten Substituenten aufweisen. Gegebenenfalls wie angegeben substituierte Phenylreste R sind unsubstituiert oder vorzugsweise durch Niederalkyl, Niederalkoxy, Halogenniederalkoxy, Niederalkenyloxy, Halogen, Trifluormethyl, Cyano, Benzylthio, Benzyloxy oder Nitro mono- oder disubstituiert, vorzugsweise in 2- und/oder 3-Stellung. Pyridyl- bzw. 1-Oxidopyridylreste R sind beispielsweise unsubstituierte oder insbesondere durch Niederalky, Niederalkoxy, gegebenenfalls S-oxidiertes Niederalkylthio oder Halogen monosubstituierte, vorzugsweise 2-substituierte 3-(1-Oxido)pyridylreste. Benzofurazanylreste und (1,3-Dioxa)indanylreste R sind vorzugsweise unsubstituiert und bedeuten z.B. 8-Benzofurazanyl bzw. 4-(1,3-Dioxa)indanyl. Phenylreste $R_1$ sind unsubstituierte oder beispielsweise durch Niederalkyl, Niederalkoxyniederalkyl, Niederalkoxyniederalkoxyniederalkyl, Cycloalkylniederalkoxyniederalkyl, Carbamylniederalkyl, Niederalkanoylaminoniederalkyl, Niederalkoxy, Niederalkenyloxy, Niederalkinyloxy, Niederalkoxyniederalkoxy, Carbamylniederalkoxy, Niederalkanoylaminoniederalkoxy, oder Cyano monosubstituiert. Phenylenreste Ph als Bestandteil der Gruppe A sind vorzugsweise unsubstituiert. Indolylreste $R_1$ sind unsubstituierte oder vorzugsweise durch Niederalkyl monosubstituierte, insbesondere 2-substituierte Indolylreste, insbesondere 4-Indolylreste.

Vor- und nachstehend sind unter "niederen" organischen Resten beispielsweise solche zu verstehen, die pro "niedere" Teilstruktur bis und mit 7, insbesondere bis und mit 4, Kohlenstoffatome (C-Atome) aufweisen.

Niederalkoxycarbonyl ist beispielsweise $C_1$-$C_4$-Alkoxycarbonyl, wie Methoxy-, Aethoxy-, Propyloxy-, Isopropyloxy-, Butyloxy-, Isobutyloxy-, Sekundärbutyloxy- oder Tertiärbutyloxycarbonyl, kann aber auch eine

$C_5$-$C_7$-Alkoxy-, wie Pentyloxy-, Hexyloxy- oder Heptyloxygruppe sein.

Niederalkansulfonyl ist beispielsweise $C_1$-$C_4$-Alkansulfonyl, insbesondere Methansulfonyl, ferner Propan-1-oder Propan-2-sulfonyl, Butansulfonyl oder, im Falle einer Gruppe Ac, 2-Methylpropan-2-sulfonyl.

Niederalkanoyl ist beispielsweise $C_1$-$C_7$-, insbesondere $C_2$-$C_5$-Alkanoyl, wie insbesondere Acetyl, Propionyl, Butyryl, Isobutyryl, Valeroyl, Isovaleroyl oder Pivaloyl, kann aber auch Formyl oder eine Hexanoyl- oder Heptanoylgruppe sein.

Niederalkylimino ist beispielsweise $C_1$-$C_4$-Alkylimino, wie Methylimino.

Niederalkyl ist beispielsweise $C_1$-$C_4$-Alkyl, wie Methyl, Aethyl, Propyl, Isopropyl, Butyl, Isobutyl, Sekundärbutyl oder Tertiärbutyl, kann aber auch eine $C_5$-$C_7$-Alkyl-, wie Pentyl-, Hexyl- oder Heptylgruppe sein.

Niederalkoxy ist beispielsweise $C_1$-$C_4$-Alkoxy, wie Methoxy, Aethoxy, Propyloxy, Isopropyloxy oder Butyloxy, ferner Isobutyloxy, Sekundärbutyloxy oder Tertiärbutyloxy.

Halogen ist insbesondere Halogen mit Atomnummer bis und mit 35, wie Fluor, Chlor oder Brom, ferner Jod.

Halogenniederalkoxy ist beispielsweise Halogen-, Dihalogen-, Trihalogen- oder Tetrahalogen-$C_1$-$C_4$-alkoxy, worin als Halogensubstituenten insbesondere gleiche oder verschiedene Halogenatome der Atomnummer bis und mit 35, wie Chlor oder vor allem Fluor, in Betracht kommen, wie Difluormethoxy oder 1,1,2-Trifluor-2-chloräthoxy.

Niederalkenyloxy ist beispielsweise $C_2$-$C_4$-Alkenyloxy, wie Allyloxy oder Methallyloxy.

Niederalkoxyniederalkyl ist beispielsweise $C_1$-$C_4$-Alkoxy-$C_1$-$C_4$-Alkoxy-$C_1$-$C_4$-alkyl, z.B. 2-Methoxyäthyl, 2-Aethoxyäthyl oder 2-Isopropyloxyäthyl. Ebenso ist Niederalkoxyniederalkoxy beispielsweise $\omega$-$C_1$-$C_4$-Alkoxy-$C_2$-$C_4$-alkoxy z.B. 2-Methoxyäthoxy, 2-Aethoxyäthoxy oder 2-Isopropyloxyäthoxy.

Niederalkoxyniederalkoxyniederalkyl trägt die terminale Niederalkoxygruppe in höherer als der $\alpha$-Stellung und ist beispielsweise entsprechendes $C_1$-$C_4$-Alkoxy-$C_2$-$C_4$-alkoxy-$C_1$-$C_4$-alkyl, wie 2-Methoxyäthoxymethyl oder 2-Isopropyloxyäthoxymethyl.

Cycloalkylniederalkoxyniederalkyl trägt die Cycloalkylniederalkoxygruppe in höherer als der $\alpha$-Stellung und ist beispielsweise Cycloalkyl-$C_1$-$C_4$-alkoxy-$C_1$-$C_4$-alkyl mit 3 bis und mit 5 Ringgliedern im Cycloalkylteil, wie 2-Cyclopropylmethoxyäthyl.

Carbamylniederalkyl ist beispielsweise Carbamyl-$C_1$-$C_4$-alkyl, wie Carbamylmethyl. Entsprechend ist Carbamylniederalkoxy beispielsweise Carbamyl-$C_1$-$C_4$-alkoxy, wie Carbamylmethoxy.

Niederalkanoylaminoniederalkyl ist beispielsweise $C_2$-$C_7$-Alkanoyl-, insbesondere $C_2$-$C_4$-Alkanoylamino-$C_1$-$C_4$-alkyl, wie Acetylaminomethyl oder 2-Acetylaminoäthyl.

Niederalkinyloxy ist beispielsweise $C_2$-$C_4$-Alkenyloxy, wie Propargyloxy.

Carbamylniederalkyl ist beispielsweise Carbamyl-$C_1$-$C_4$-alkyl, wie Carbamylmethyl. Entsprechend ist Carbamylniederalkoxy beispielsweise Carbamyl-$C_1$-$C_4$-alkoxy, wie Carbamylmethoxy.

Niederalkanoylaminoniederalkoxy ist beispielsweise $\omega$-$C_2$-$C_7$-Alkanoyl-, insbesondere $C_2$-$C_4$-Alkanoylamino-$C_2$-$C_4$-alkoxy, wie 2-Acetylaminoäthoxy.

Hydroxyniederalkyl ist beispielsweise Hydroxy-$C_1$-$C_4$-alkyl, wie Hydroxymethyl oder 2-Hydroxyethyl.

Niederalkylenoxyphenylenoxyniederalkylenreste sind beispielsweise 10- bis und mit 14-gliedrige $c_2$-$C_4$-Alkylenoxy-phenylenoxy-$C_2$-$C_4$-alkylenreste, z.B. der Formel -$alk_1$-O-Ph-O-$alk_3$-, worin $alk_1$ und $alk_3$ gleiche oder verschiedene $C_2$-$C_4$-Alkylenreste der vorstehend definierten Art bedeuten und Ph für Phenylen, insbesondere p-Phenylen, steht, wie Aethylenoxyphenylenoxyäthylen, Aethylenoxyphenylenoxy-1,2-propylen oder Aethylenoxyphenylenoxy-1,2-(2-methyl)propylen, ferner 1,3-Propylenoxyphenylenoxyäthylen.

Niederalkylenphenylenoxyniederalkylenreste sind beispielsweise 8-bis und mit 14-gliedrige $C_1$-$C_4$-Alkylenphenylenoxy-$C_2$-$C_4$-alkylen-bzw. $C_2$-$C_4$-Alkylenoxyphenylen-$C_1$-$C_4$-alkylenreste, z.B. der Formel -$alk_1$-O-Ph-$alk_2$-, worin $alk_1$ $C_2$-$C_4$-Alkylen der vorstehend definierten Art, $alk_2$ $C_2$-$C_4$-Alkylen oder $C_1$-$C_4$-Alkyliden der vorstehend definierten Art und Ph Phenylen, insbesondere p-Phenylen, bedeutet, wie Aethylenoxyphenylenäthylen.

Niederalkylidenphenylenoxyniederalkylen ist z.B. der Formel -$alk_2$-Ph-O-$alk_3$-, wie $C_1$-$C_4$-Alkyliden-phenylenoxy-$C_2$-$C_4$-alkylen, z.B. Methylen-, Ethyliden- oder Isopropyliden-1,4-phenylenoxyethylen.

Niederalkylen ist beispielsweise $C_2$-$C_4$-Alkylen, dessen freie Valenzen von benachbarten oder zueinander 1,3- oder 1,4-ständigen C-Atomen ausgehen und bedeutet als $alk_1$ z.B. Aethylen oder in zweiter Linie 1,3-Propylen oder 1,4-Butylen und als $alk_3$ z.B. Aethylen, 1,2-Propylen oder 1,2-(2-Methyl)propylen.

Niederalkyliden $alk_2$ ist beispielsweise $C_1$-$C_4$-Alkyliden, dessen freie Valenzen vom gleichen C-Atom ausgehen; und bedeutet beispielsweise Methylen, Aethyliden, Propyliden oder Isopropyliden.

Pyridyl ist 2-, 3- und 4-Pyridyl, 1-Oxido-pyridyl ist 1-Oxido-2-pyridyl, -3-pyridyl und -4-pyridyl, während Benzofurazanyl (auch 2,1,3-Benzooxadiazolyl) in erster Linie 4-Benzofurazanyl und Indolyl z.B. 2-, 3- und insbesondere 4-Indolyl bedeutet.

Gegebenenfalls S-oxidiertes Niederalkylthio bedeutet Niederalkylthio, Niederalkansulfinyl oder Niederalkansulfonyl.

Niederalkylthio ist beispielsweise $C_1$-$C_4$-Alkylthio, insbesondere Methylthio, ferner Aethylthio, Propylthio, Isopropylthio oder Butylthio.

Niederalkansulfinyl ist beispielsweise $C_1$-$C_4$-Alkansulfinyl, insbesondere Methansulfinyl, ferner Propan-1-oder Propan-2-sulfinyl oder Butansulfinyl.

Die Verbindungen der Formel I können in Form von Salzen, insbesondere von Säureadditionssalzen, in erster Linie entsprechenden pharmazeutisch verwendbaren, nicht-toxischen Säureadditionssalzen, vorliegen. Solche Salze sind z.B. diejenigen mit Halogenwasserstoffsäuren, z.B. Chlorwasserstoffsäure oder

Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Benzolsulfonsäure, 4-Methyl-benzolsulfonsäure oder Naphthalin-2-sulfonsäure, oder mit anderen sauren organischen Substanzen, wie Ascorbinsäure. Entsprechende Säureadditionssalze werden bevorzugt mit dem basischen Zentrum der Seitenkette gebildet. Umfasst sind ferner für pharmazeutische Verwendungen nicht geeignete Salze, da diese beispielsweise für die Isolierung bzw. Reinigung freier erfindungsgemässer Verbindungen oder anderer pharmazeutisch verwendbarer Salze verwendet werden können.

Die Verbindungen der Formel I und deren Salze besitzen wertvolle pharmakologische Eigenschaften, vor allem im cardiovasculären Bereich. Sie wirken gleichzeitig als Calcium-Antagonisten und β-Rezeptoren-Blokker.

So besitzen sie eine ausgeprägte Fähigkeit zur Bindung an Calcium-Kanälen. Diese Eigenschaft lässt sich in vitro bei Konzentrationen von etwa 1 bis etwa 8 nMol/l als Verdrängung von $^3$H-Nitrendipin aus seinen Bindungsstellen an Meerschweinchen-Myokardmembranen analog der Methodik von Erne, P. et al., Biochem. Biophys. Res. Com. 118, 842 (1984) nachweisen.

Weiterhin weisen die Verbindungen der Formel I gleichzeitig die Fähigkeit zur Bindung an β-Rezeptoren auf. Diese Eigenschaft lässt sich in vitro bei Konzentrationen von etwa 2 bis etwa 250 nMol/l als Verdrängung von $^3$H-Dihydroalprenolol aus seinen Bindungsstellen an Ratten-Hirn-Membranen, analog der Methodik Bylund und Snyder, Mol. Pharmacol 12, 568 (1976) nachweisen, wobei besonders hervorzuheben ist, dass, wenn in der gleichen Versuchsanordnung die Affinität zu den $\beta_1$- und $\beta_2$-Rezeptoren separat bestimmt wird, ein Teil der Verbindungen eine ausgeprägte $\beta_1$-Selektivität aufweisen ($\beta_2/\beta_1$-Verhältnis etwa 30 bis 100).

In einem weiteren pharmakologischen Testmodell, in dem der Einfluss der Verbindungen auf pressorische und tachykarde Effekte, induziert durch diverse Agonisten (z.B. Angiotenzin II, Noradrenalin, Adrenalin) bzw. elektrische Stimulation des Sympathikus bei der despinalisierten Ratte untersucht wird, wurde beispielsweise in einem Dosis bereich von etwa 10 bis etwa 30 mg/kg p.o. beobachtet, dass die Verbindungen der Formel I einerseits die Blutdruckanstiege (als Ausdruck der Calcium-antagonistischen Wirkung) und andererseits die Herzfrequenzzunahmen (als Ausdruck der β-blockierenden Eigenschaft) in ähnlichem Umfang hemmen.

Dementsprechend können die Verbindungen der Formel I und ihre pharmazeutisch verwendbaren Salze z.B. als Pharmazeutika, generell bei den für Calcium-Antagonisten und β-Blockern bekannten Indikationen verwendet werden, insbesondere bei der Behandlung von cardiovaskulären Krankheitszuständen, wie Hypertonie und Angina pectoris. Ein weiterer Gegenstand der Erfindung ist die Verwendung der erfindungsgemässen Verbindungen zur Herstellung von Arzneimitteln, insbesondere von Antihypertonika, Antianginosa und Antiarrhythmika, zur therapeutischen und prophylaktischen Behandlung des menschlichen, ferner tierischen Körpers. Dabei kann auch die gewerbsmässige Herrichtung der Wirksubstanzen eingeschlossen sein.

Die Erfindung betrifft in erster Linie Verbindungen der Formel I, worin Ac Niederalkoxycarbonyl, Niederalkanoyl oder Niederalkansulfonyl bedeutet, X Oxy, Imino oder Niederalkylimino darstellt, A einen durch eine Gruppe der Formel $-X_1-Ph-X_2$, worin mindestens einer der Reste $X_1$ und $X_2$ Oxy und ein davon verschiedener Rest $X_1$ oder $X_2$ eine direkte Bindung darstellt und Ph einen unsubstituierten oder durch Niederalkyl, Niederalkoxy, Halogen, Trifluormethyl und/oder Nitro substituierten Phenylenrest bedeutet, unterbrochenen Alkylenrest darstellt, R einen gegebenenfalls durch Niederalkyl, Niederalkoxy, Halogenniederalkoxy, Niederalkenyloxy, Halogen, Trifluormethyl, Cyano, einen gegebenenfalls durch Niederalkyl, Niederalkoxy, Halogen, Trifluormethyl und/oder Nitro substituierten Benzylthio- bzw. Benzyloxyrest und/oder Nitro substituierten Phenylrest, einen gegebenenfalls durch Niederalkyl, Niederalkoxy, gegebenenfalls S-oxidiertes Niederalkylthio und/oder Halogen substituierten, gegebenenfalls 1-oxidierten Pyridylrest oder einen (1,3-Dioxa)indanyl- oder Benzofurazanylrest bedeutet, $R_1$ einen gegebenenfalls durch Niederalkyl, Niederalkoxyniederalkyl, Niederalkoxyniederalkoxyniederalkyl, Cycloalkylniederalkoxyniederalkyl, Carbamylniederalkyl, Niederalkanoylaminoniederalkyl, Niederalkoxy, Niederalkenyloxy, Niederalkinyloxy, Niederalkoxyniederalkoxy, Carbamylniederalkoxy, Niederalkanoylaminoniederalkoxy, und/oder Cyano substituierten Phenylrest oder einen gegebenenfalls durch Niederalkyl substituierten Indolylrest bedeutet, $R_2$ für Niederalkyl steht und $R_3$ Niederalkyl, Hydroxyniederalkyl, Cyano oder Amino bedeutet, und ihre Säureadditionssalze.

Die Erfindung betrifft insbesondere Verbindungen der Formel I, worin Ac $C_1$-$C_7$-Alkanoyl, $C_1$-$C_7$-Alkoxycarbonyl oder $C_1$-$C_4$-Alkansulfonyl bedeutet, X für Oxy, Imino oder $C_1$-$C_4$-Alkylimino steht, A einen im Phenylenteil gegebenenfalls durch $C_1$-$C_4$-Alkyl, $C_1$-$C_4$-Alkoxy, Halogen, Trifluormethyl und/oder Nitro substituierten 8- bis und mit 14-gliedrigen $C_1$-$C_4$-Alkylenphenylenoxy-$C_2$-$C_4$-alkylenrest bzw. $C_2$-$C_4$-Alkylenoxyphenylen-$C_1$-$C_4$-alkylenrest oder 10- bis und mit 14-gliedrigen $C_1$-$C_4$-Alkylenoxyphenylenoxy-$C_1$-$C_4$-alkylenrest bedeutet, R einen gegebenenfalls durch $C_1$-$C_4$-Alkyl, $C_1$-$C_4$-Alkoxy, Mono- bis und mit Tetrahalogen-$C_1$-$C_4$-alkoxy, $C_2$-$C_4$-Alkenyloxy, einen gegebenenfalls durch $C_1$-$C_4$-Alkyl, $C_1$-$C_4$-Alkoxy, Halogen, Trifluormethyl und/oder Nitro substituiertes Benzylthio- bzw. Benzyloxyrest, Halogen, Trifluormethyl, Cyano und/oder Nitro substituierten Phenylrest, einen gegebenenfalls durch $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl und/oder Halogen substituierten Pyridyl- oder 1-Oxidopyridyl-,

vorzugsweise 3-Pyridyl- oder 3-(1-Oxido)pyridylrest oder einen 4-(1,3-Dioxa)indanyl-oder 8-Benzfurazanylrest darstellt, $R_1$ einen gegebenenfalls durch $C_1$-$C_4$-Alkyl, $C_1$-$C_4$-Alkoxy-$C_2$-$C_4$-alkyl, $C_1$-$C_4$-Alkoxy-$C_2$-$C_4$-alkoxy-$C_1$-$C_4$-alkyl, 3-bis 5-gliedriges Cycloalkyl-$C_1$-$C_4$-alkoxy-$C_2$-$C_4$-alkyl, Carbamyl-$C_1$-$C_4$-alkyl, $C_2$-$C_7$-Alkanoylamino-$C_1$-$C_4$-alkyl, $C_1$-$C_4$-Alkoxy, $C_2$-$C_4$-Alkenyloxy, $C_2$-$C_4$-Alkinyloxy, ω-$C_1$-$C_4$-Alkoxy-$C_2$-$C_4$-alkoxy, Carbamyl-$C_1$-$C_4$-alkoxy, ω-$C_2$-$C_7$-Alkanoylamino-$C_2$-$C_4$-alkoxy und/oder Cyano substituierten Phenylrest oder gegebenenfalls durch $C_1$-$C_4$-Alkyl substituierten Indolylrest bedeutet, $R_2$ $C_1$-$C_4$-Alkyl darstellt und $R_3$ $C_1$-$C_4$-Alkyl, Hydroxy-$C_1$-$C_4$-alkyl, Cyano oder Amino bedeutet, und ihre Säureadditionssalze.

Die Erfindung betrifft vor allem Verbindungen der Formel I, worin Ac $C_1$-$C_4$-Alkoxycarbonyl, wie Methoxycarbonyl, bedeutet, X für Oxy steht, -X-A- einen Rest der Formeln -X-alk$_1$-O-Ph-alk$_2$- oder -X-alk$_1$-O-Ph-O-alk$_3$-, worin alk$_1$ und alk$_2$ gleiche oder verschiedene $C_2$-$C_4$-Alkylenreste bedeuten, wobei alk$_1$ z.B. für Aethylen und alk$_3$ z.B. für Aethylen, 1,2-Propylen oder 1,2-(2-Methyl)propylen steht, und alk$_2$ $C_2$-$C_4$-Alkylen, z.B. Aethylen oder 1,3-Propylen, oder $C_1$-$C_4$-Alkyliden, z.B. Methylen oder Aethyliden, bedeutet, wie 2-(p-Aethylenphenoxy)äthoxy, 2-(p-Aethylenoxyphenyloxy)äthoxy, 2-(p-Aethylenphenoxy)äthylen, 2-[p-(2-Propylenoxy)phenoxy)äthoxy oder 2-{p-[2-(2-Methylpropylenoxy)phenoxy}äthoxy, R für unsubstituiertes oder vorzugsweise in 2- und/oder 3-Stellung durch Halogen der Atomnummer bis und mit 35, wie Chlor, mono- oder disubstituiertes oder durch Trifluormethyl, Nitro oder Cyano monosubstituiertes Phenyl bedeutet, $R_1$ gegebenenfalls durch $C_1$-$C_4$-Alkoxy-$C_2$-$C_4$-alkoxy, wie 2-Methoxyäthoxy, $C_1$-$C_4$-Alkoxy-$C_1$-$C_4$-alkyl, wie 2-Methoxyäthyl, $C_1$-$C_4$-Alkoxy-$C_2$-$C_4$-alkoxy-$C_1$-$C_4$-alkyl, wie 2-Isopropyloxyäthoxymethyl, 3-bis 5-gliedriges Cycloalkyl-$C_1$-$C_4$-alkoxy-$C_2$-$C_4$-alkyl, wie 2-Cyclopropylmethoxyäthyl, Carbamyl-$C_1$-$C_4$-alkyl, wie Carbamylmethyl oder Cyano monosubstituierten Phenyl bedeutet und $R_2$ und $R_3$ $C_1$-$C_4$-Alkyl, wie Methyl, bedeuten, und ihre Säureadditionssalze.

Die Erfindung betrifft vor allem Verbindungen der Formel I, worin $R_1$ Phenyl, 2-Methyl, 4-(2-Methoxyäthyl)-, 4-(2-Isopropyloxyäthoxymethyl)-, 4-(2-Methoxyäthoxy)-4-(2-Isopropyloxy-äthoxy)-, 4-(2-Cycloproylmethoxyäthyl)-, 4-Carbamoylmethyl-, 2-Methoxy-, 2-Isopropoxy-, 2-Cyano-phenyl, 4-Indolyl oder 2-Methyl-4-indolyl bedeutet.

Die Erfindung betrifft vor allem Verbindungen der Formel I, worin Ac $C_1$-$C_4$-Alkoxy-carbonyl, wie Methoxycarbonyl bedeutet, -X-A- für eine Gruppe der Formel -X-alk$_1$-O-Ph-alk$_3$- oder X-alk$_1$-O-Ph-O-alk$_3$ steht, wobei X Oxy bedeutet, alk$_1$ und alk$_3$ unabhängig voneinander $C_2$-$C_4$-Alkylen, wie Ethylen, 1,2-, 1,3-Propylen oder 1,2-(2-Methyl)propylen, bedeuten und Ph 1,2-, 1,3- oder 1,4-Phenylen bedeutet, R für in 2- und/oder 3-Stellung durch Halogen der Atomnummer bis und mit 35, wie Chlor, mono- oder disubstituierten, durch $C_1$-$C_4$-Alkoxy, wie Methoxy, insbesondere in Position 3, oder durch Nitro, insbesondere in Position 2 oder 3, monosubstituiertes Phenyl bedeutet, $R_1$ durch 2-Methoxyäthyl, 2-Isopropyloxy-äthoxymethyl, 2-Cyclopropylmethoxy-äthyl, 2-Methoxyäthoxy oder 2-Isopropyloxy-äthoxy, insbesondere jeweils in 4-Stellung, monosubstituiertes oder durch Cyano, insbesondere in 2-Stellung, monosubstituiertes Phenyl bedeutet, und $R_2$ und $R_3$ jeweils $C_1$-$C_4$-Alkyl, wie Methyl, bedeuten und ihre Säureadditionssalze.

Die Erfindung betrifft vor allem Verbindungen der Formel I, worin Ac $C_1$-$C_4$-Alkoxy-carbonyl, wie Methoxycarbonyl, bedeutet, -X-A- für eine Gruppe der Formel -X-alk$_1$-O-Ph-alk$_3$- oder -X-alk$_1$-O-Ph-O-alk$_3$ steht, wobei X Oxy ist, alk$_1$ und alk$_3$ unabhängig voneinander $C_2$-$C_4$-Alkylen, wie Ethylen, 1,2-, 1,3-Propylen oder 1,2-(2-Methyl)-propylen, bedeuten und Ph 1,4-, ferner 1,2- und 1,3-Phenylen bedeutet, R für 3-Nitro- oder 3-Methoxyphenyl steht, $R_1$ 4-(2-Methoxyäthyl)-, 4-(2-Isopropyloxy-äthoxymethyl)-, 4-(2-Cyclopropylmethoxy-äthyl)-, 4-(2-Methoxyäthoxy)-4-(2-Isopropyloxy-äthoxy)-phenyl oder 2-Cyanophenyl bedeutet und $R_2$ und $R_3$ jeweils $C_1$-$C_4$-Alkyl, wie Methyl, bedeuten, und ihre Säureadditionssalze.

Die Erfindung betrifft vor allem Verbindungen der Formel I, worin Ac $C_1$-$C_3$-Alkoxy-carbonyl, wie Methoxycarbonyl, bedeutet, -X-A- für eine Gruppe der Formel -X-$C_2H_4$-O-Ph-alk$_3$- oder -X-$C_2H_4$-O-Ph-O-alk$_3$-steht, wobei X Oxy ist, Ph 1,4- oder 1,2-Phenylen bedeutet uns alk$_3$ $C_2$-$C_4$-Alkylen, wie Ethylen oder 1,2-(2-Methyl)-propylen, bedeutet, R 3-Nitro- oder 3-Methoxyphenyl bedeutet, $R_2$ und $R_3$ $C_1$-$C_2$-Alkyl, wie Methyl, bedeuten und $R_1$ 4-(2-Methoxyäthyl)-, 4-(2-Isopropyloxy-äthoxymethyl)-, 4-(2-Cyclopropylmethoxy-äthyl)-, oder 4-(2-Methoxy-äthoxy)-phenyl oder $R_1$ 2-Cyanophenyl bedeutet, und ihre Säureadditionssalze.

Die Erfindung betrifft vor allem Verbindungen der Formel I, worin Ac $C_1$-$C_3$-Alkoxycarbonyl, wie Methoxycarbonyl, bedeutet, -X-A- für eine Gruppe der Formel -X-$C_2H_5$-O-Ph-O-alk$_3$ steht, wobei X Oxy ist, Ph 1,4-Phenylen bedeutet und alk$_3$ Ethylen oder 1,2-(2-Methyl)-propylen bedeutet, R 3-Nitrophenyl bedeutet, $R_1$ 4-(2-Methoxyäthyl)- oder 4-(2-Isopropyloxy-äthoxymethyl)-phenyl bedeutet und $R_2$ und $R_3$ $C_1$-$C_2$-Alkyl, wie Methyl, bedeuten, und ihre Säureadditionssalze.

Die pharmakologischen Eigenschaften sind besonders günstig, wenn des asymmetrische C-Atom der Gruppe -NH-$CH_2$-CHOH-$CH_2$-O$R_1$ gemäss der Regel von Cahn, Ingold und Prelog in der S-Konfiguration vorliegt:

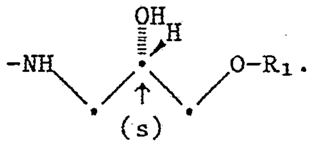

Die Erfindung betrifft namentlich die in den Beispielen genannten Verbindungen der Formel I und ihre Säureadditionssalze sowie Verfahren ihrer Herstellung.

Die Erfindung betrifft ferner ein auf an sich bekannten Methoden beruhendes Verfahren zur Herstellung von Verbindungen der Formel I und ihrer Säureadditionssalze. Das erfindungsgemässe Verfahren ist dadurch gekennzeichnet, dass man

a) eine Verbindung der Formel

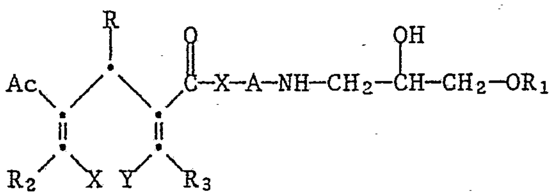 (II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

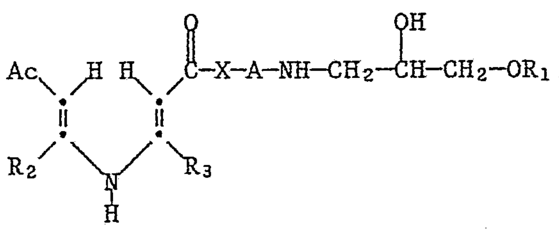 (IV),

oder einem Tautomeren davon oder einem entsprechenden Tautomerengemisch umsetzt, oder

c) in einer Verbindung der Formel

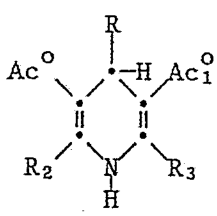 (V),

in welcher $Ac^o$ eine Gruppe -Ac oder einen in diesen überführbaren Rest und $Ac_1^o$ einen in eine Gruppe der Formel

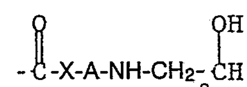 (VI) überführbaren Rest oder $Ac^o$ einen in eine Gruppe Ac überführbaren Rest und $Ac_1^o$ eine Gruppe der Formel VI bedeutet, $Ac^o$ in eine Gruppe Ac und/oder $Ac_1^o$ in eine Gruppe der Formel VI überführt, oder

d) eine Verbindung der Formel

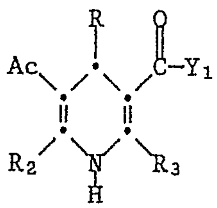

(VII)

mit einer Verbindung der Formel

$$Y_2\text{-NH-}CH_2\text{-}\overset{OH}{\overset{|}{CH}}\text{-}CH_2\text{-}OR_1$$

(VIII) umsetzt, worin $Y_1$ eine Gruppe Y und $Y_2$ eine Gruppe der Formel H-X-A-oder $Y_1$ eine Gruppe der Formel -X-A-Y und $Y_2$ Wasserstoff oder $Y_1$ eine Gruppe der Formel -X-H und $Y_2$ eine Gruppe der Formel Y-A-bedeutet und Y jeweils einen abspaltbaren Rest darstellt, oder

e) eine Verbindung der Formel

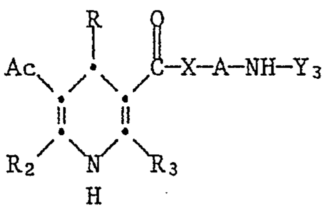

(IX)

mit einer Verbindung der Formel

$Y_4\text{-}OR_1$ (X)

umsetzt, worin einer der Reste $Y_3$ und $Y_4$ Wasserstoff und der andere eine Gruppe der Formel

$$\text{-}CH_2\text{-}\overset{Y_5}{\overset{|}{CH}}\text{-}CH_2\text{-}Y_6$$

(XI)

bedeutet, in der $Y_5$ für Hydroxy und $Y_6$ für einen abspaltbaren Rest steht oder $Y_5$ und $Y_6$ gemeinsam eine Epoxygruppe darstellen, und. wenn erwünscht, eine erhaltene Verbindung der Formel I in eine andere Verbindung der Formel I unwandelt, und/oder eine erhaltene freie Verbindung der Formel I in ein Salz oder ein erhaltenes Salz in die freie Verbindung oder in ein anderes Salz überführt, umwandelt und/oder, wenn erwünscht, ein erhaltenes Isomerengemisch in die individuellen Isomere auftrennt.

Ueblicherweise werden die in der Verfahrensvariante a) verwendeten Ausgangsstoffe der Formel II In situ gebildet, so dass der verfahrensgemässe Ringschluss unter den Reaktionsbedingungen für die Herstellung des Ausgangsmaterials stattfindet. So kann man die Ausgangsstoffe der Formel II und unter den Reaktionsbedingungen üblicherweise auch die entsprechenden Endprodukte der Formel I erhalten, indem man

aa) eine Verbindung der Formel R-CHO (III) oder ein reaktionsfähiges funktionelles Derivat davon, mit einer Verbindung der Formel

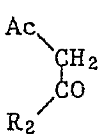

(IIa)

und einer Verbindung der Formel

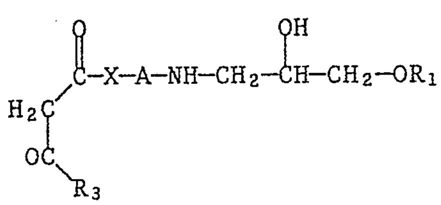

(IIb)

und Ammoniak umsetzt, oder

ab) eine Verbindung der Formel III oder ein reaktionsfähiges funktionelles Derivat davon mit einer

Verbindung der Formel

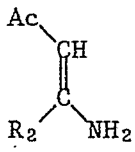

(IIc),

und einer Verbindung der Formel IIb umsetzt, oder

ac) eine Verbindung der Formel III oder ein reaktionsfähiges funktionelles Derivat davon mit einer Verbindung der Formel

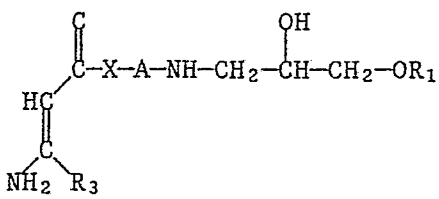

(IId),

und einer Verbindung der Formel IIa oder IIc umsetzt, oder

ad) Ammoniak mit einer Verbindung der Formel

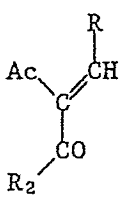

(IIe)

und einer Verbindung der Formel IIb umsetzt, oder

ae) Ammoniak mit einer Verbindung der Formel

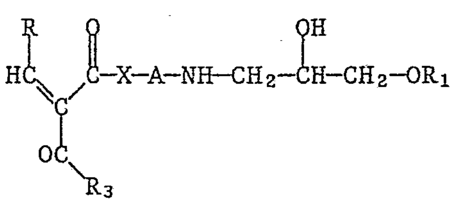

(IIf)

und einer Verbindung der Formel IIa oder IIc umsetzt, oder

af) Ammoniak mit einer Verbindung der Formel

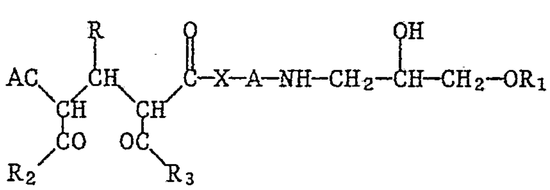

(IIg)

umsetzt. Dabei können mit Ausnahme der Verbindung der Formel III die Verbindungen der Formeln IIa bis IIg in Form von Tautomeren oder in Form von Tautomerengemischen verwendet werden. Ausgangsstoffe der obigen Formeln mit salzbildenden Eigenschaften können auch in Form von Salzen verwendet werden. Ferner haben in den obgenannten Verbindungen die Gruppen R, $R_1$, $R_2$, $R_3$, X, Ac und A die im Zusammenhang mit der Formel I gegebenen Bedeutungen.

Reaktionsfähige funktionelle Derivate des Aldehyds der Formel III sind u.a. die entsprechenden Acetale, d.h. die der Gruppe R entsprechenden verätherte Dihydroxymethyl-Verbindungen, wie Diniederalkyl-, z.B.

Dimethyl- oder Diäthylacetale, Acylale, z.B. die entsprechenden Diacyloxymethyl- oder Dihalogenmethyl-Verbindungen, wie Diniederalkanoylacylale, z.B. Diacetylacylale, oder die entsprechenden Dihalogen-, z.B. Dichlor- oder Dibrom-Verbindungen, ferner Additionsverbindungen, wie solche mit einem Alkalimetall-, z.B. Kaliumhydrogensulfit.

Das für die vorstehend beschriebenen Ringschlussreaktionen verwendete Ammoniak kann in Form eines diese Verbindung in situ abgebenden Mittels, z.B. in Form eines Ammoniumsalzes, wie Ammoniumacetat oder Ammoniumhydrogencarbonat, verwendet werden.

Bei der Ringschlussreaktion a) und den Kondensationsreaktionen aa) bis af) zur Herstellung des üblicherweise in situ gebildeten Ausgangsmaterials für die Ringschlussreaktion handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Zwischenprodukten. Bei der Umsetzung von Verbindungen der Formel III mit Verbindungen der Formeln IId und IIc gemäss der Stufe ac) wird zusätzlich zu bzw. anstelle von Wasser Ammoniak abgespalten. Falls nach der Variante (aa) Verbindungen der Formel I hergestellt werden sollen, in denen sich $R_2$ und $R_3$ voneinander unterscheiden, können Nebenprodukte entstehen, die in 2- und 6-Stellung die gleichen Substituenten enthalten. Durch nicht-gleichzeitiges Zusammengeben der Reaktionsteilnehmer kann die Bildung solcher Nebenprodukte weitgehend vermieden werden, indem man einen bestimmten Reaktionsverlauf fördert, der in situ gemäss einer andern Variante verläuft, da entsprechend der gestaffelten Zugabe der Reaktionskomponenten z.B. zunächst eine Verbindung der allgemeinen Formel IIc oder der Formel IId entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, und/oder eines geeigneten Dehydratisierungs- oder Wasser-aufnehmenden Mittels, ferner üblicherweise in Gegenwart eines inerten organischen Lösungsmittels und bei Reaktionstemperaturen beispielsweise im Bereiche von etwa Raumtemperatur bis etwa 150°C, insbesondere bei Siedetemperatur des Lösungsmittels. Gegebenenfalls erfolgt die Umsetzumg in einer Inertgas-, z.B. Stickstoffatmosphäre, und/oder, z.B. bei Verwendung eines niedrigsiedenden Lösungsmittels und/oder von Ammoniak, im geschlossenen Gefäss unter erhöhtem Druck.

Die in den Verfahrensvarianten verwendeten Ausgangsstoffe sind bekannt oder können nach an sich bekannten Verfahren hergestellt werden.

So lassen sich z.B. Ausgangsstoffe der Formel IIb durch Umsetzung von Diketen oder Verbindungen der Formel

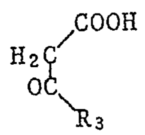

(IIba)

mit Verbindungen der Formel

OH
H-X-A-NH-$CH_2$-CH-$CH_2$-$OR_1$ (IIbb) auf übliche Weise herstellen. Anstelle von Carbonsäuren der Formel IIba können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanomethylester, verwendet werden. Diese können, gegebenenfalls in Gegenwart von Kondensationsmitteln, durch Umsetzung mit einer Verbindung der Formel IIbb, freie Carbonsäuren der Formel IIba auch durch Umsetzen mit Verbindungen der Formel IIbb, worin anstelle der Gruppe HX- die Azidogruppe steht, zu Verbindungen der Formel IIb umgesetzt werden. Carbonsäuren der Formel IIba können auch als Salze, insbesondere Alkalimetall-oder Erdalkalimetallsalze mit reaktionsfähigen Estern von Alkoholen der Formel IIbb, worin X für Sauerstoff steht, wie entsprechenden Halogeniden, z.B. Chloriden, Bromiden oder Jodiden, oder organischen Sulfonsäureestern, z.B. Niederalkansulfonsäure- oder Arensulfonsäureestern, wie Methansulfonsäure- bzw. p-Toluolsulfonsäureestern, zu entsprechenden Carbonsäureestern umgesetzt werden, oder man hydrolysiert entsprechende hydrolysierbare Iminoester, wie entsprechende Iminoniederalkylester, zu den Estern. Iminoester dieser Art sind beispielsweise aus den Verbindungen der Formel IIba entsprechenden Nitrilen der Formel

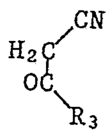

(IIbc)

durch Umsetzung mit Verbindungen der Formel IIbb, worin X für Sauerstoff steht, in Gegenwart eines sauren Kondensationsmittels, etwa Chlorwasserstoff, in einem geeigneten Lösungsmittel, etwa eines solchen inerten Charakters, wie eines Aromaten, z.B. Benzol, auf übliche Weise zugänglich.

Verbindungen der Formel IIbb wiederum sind auf an sich bekannte Weise zugänglich, z.B. durch Umsetzung von Verbindungen der Formel

H-X-A-Y (IIbd),

worin Y eine geeignete Abgangsgruppe, z.B. eine reaktionsfähige veresterte Hydroxygruppe, wie etwa Halogen, z.B. Chlor, Brom oder Jod, oder eine Sulfonyloxygruppe, z.B. eine Arylsulfonyloxy-, wie p-Toluolsulfonyloxygruppe, darstellt, mit Verbindungen der Formel

OH

$H_2N-CH_2-CH-CH_2-OR_1$ (IIbe),

zweckmässigerweise in Gegenwart eines basischen Kondensationsmittels, wie eines Oxids, Hydroxids oder Carbonats eines Alkalimetall- oder Erdalkalimetalls, wie Natriumhydroxid oder Calciumcarbonat, üblicherweise in Gegenwart eines Lösungsmittels, etwa eines Niederalkanols, wie Aethanol, und bei erhöhter oder erniedrigter Temperatur. Verbindungen der Formel IIbe können auch in Form ihrer Metallderivate eingesetzt werden, worin das am Stickstoff stehende Wasserstoffatom durch ein geeignetes Metall, etwa Lithium oder Kalium ersetzt ist. In solchen Fällen erfolgt die beschriebene Umsetzung mit Verbindungen der Formel IIbd in einem inerten Wasser freien Lösungsmittel, etwa eines solchen ätherartigen Charakters, wie Tetrahydrofuran, oder eines aromatischen Lösungsmittels, etwa Toluol.

Der Formel IIbe entsprechende Metallverbindungen sind in üblicher Weise, z.B. durch Umsetzung mit einer geeigneten Alkalimetall-organischen Verbindung, etwa Butyllithium, in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung, ohne diese zu isolieren, für die oben beschriebene Umsetzung verwendet werden kann.

Verbindungen der Formel IIbe wiederum sind auf an sich bekannte Weise herstellbar, beispielsweise durch Umsetzung von Epichlorhydrin zunächst mit einer Verbindung der Formel $R_1$-OH (IIbf) und dann mit Ammoniak.

Die Umsetzung von freien Carbonsäuren der Formel IIba mit Verbindungen der Formel IIbb erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N′-Diäthyl-, N,N′-Dicyclohexyl-oder N-Aethyl-N′-(3-dimethylaminopropyl)-carbodiimid, in inerten organischen Lösungsmitteln durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. Alkalimetall- oder Erdalkalimetallhydroxiden oder -carbonaten, wie Natrium-, Kalium-oder Calciumhydroxid bzw. -carbonat, mit Alkoholen oder mit Alkoholaten, z.B. Alkalimetall-niederalkanolaten, umgesetzt.

Die Umsetzungen von aktivierten Estern, z.B. Cyanmethyl- oder Pentachlorphenylestern, mit Verbindungen der Formel IIbb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoesterausgangsstoffen erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann z.B. auch aus einem Gemisch von Nitril, Alkohol und Schwefelsäure mit geeignetem Wassergehalt ohne Isolierung des in situ entstandenen Imidoesters die gewünschte Esterverbindung der Formel IIb erhalten.

Ausgangsstoffe der Formeln IId, IIf und IIg können ausgehend von entsprechenden Ausgangsmaterialien auf analoge und übliche Weise hergestellt werden.

In der Verfahrensvariante b) werden die Ausgangsstoffe der Formel IV analog wie bei der Herstellung von Ausgangsstoffen der Formel II beschrieben in situ gebildet, und der verfahrensgemässe Ringschluss zu den Endprodukten der Formel I kann unter den für die Herstellung des Ausgangsmaterials gegebenen Bedingungen im gleichen Reaktionsansatz erfolgen. Dementsprechend sind Ausgangsstoffe der Formel IV

durch Umsetzung von Verbindungen der Formel IIb mit solchen der Formel IIa und Ammoniak, oder von Verbindungen der Formel IIb mit solchen der Formel IIc, oder von Verbindungen der Formel IId mit solchen der Formel IIa oder IIc zugänglich, wobei solche Umsetzungen üblicherweise in einem geeigneten Lösungsmittel, etwa einem Nieder alkanol, wie Aethanol, gegebenenfalls bei erhöhter oder erniedrigter Temperatur, zweckmässigerweise unter einem Schutzgas, wie Stickstoff, durchgeführt werden.

Ausgangsstoffe der Formel V für die Verfahrensvariante c) sind beispielsweise solche, in denen $Ac^o$ Carboxy, anhydridisiertes Carboxy, wie Halogencarbonyl, z.B. Chlorcarbonyl, oder Azidocarbonyl und $Ac^o_1$ eine Gruppe der Formel VI bedeutet, oder worin $Ac^o$ für einen Rest Ac steht und $Ac^o_1$ eine Gruppe der Formeln

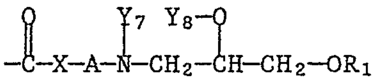 (VI')

darstellt, wobei einer der Reste $Y_7$ und $Y_8$ Wasserstoff und der andere einen einwertigen abspaltbaren, d.h. durch Wasserstoff ersetzbaren, Substituenten bedeutet oder $Y_7$ und $Y_8$ gemeinsam einen zweiwertigen abspaltbaren, d.h. durch Wasserstoff ersetzbaren, Substituenten darstellen.

Die genannten Gruppen $Ac^o$ können, gegebenenfalls in Gegenwart von Kondensationsmitteln, durch Umsetzung mit einem entsprechenden Niederalkanol oder einem reaktionsfähigen Derivat davon, z.B. einem entsprechenden Alkoholat, freie Carbonsäuren auch durch Umsetzen mit Diazoniederalkanen in Gruppen Ac überführt werden.

Die Umwandlung von Gruppen $Ac^o_1$ der Formel VI' erfolgt beispielsweise durch Abspaltung des Restes $Y_7$ bzw. $Y_8$ bzw. $Y_7 + Y_8$, d.h. Ersatz derselben durch Wasserstoff.

Die Abspaltung der Gruppe $Y_7$ bzw. $Y_7 + Y_8$ wird mittels Solvolyse, wie Hydrolyse, Alkoholyse oder Acidolyse, oder mittels Reduktion, einschliesslich Hydrogenolyse, vorgenommen.

Als abspaltbare Gruppe $Y_7$ bzw. $Y_8$ ist eine hydrogenolytisch abspaltbare α-Arylniederalkylgruppe besonders geeignet, wie eine gegebenenfalls substituierte 1-Polyphenylniederalkyl- oder 1-Phenylniederalkylgruppe, z.B. Benzhydryl oder Trityl, worin Substituenten, insbesondere des Phenylteils, z.B. Niederalkyl, wie Methyl, oder Niederalkoxy wie Methoxy, sein können, und in erster Linie Benzyl. Die Gruppe $Y_7$ bzw. $Y_8$ kann auch einen solvolytisch, wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenylniederalkylgruppe, z.B. wie oben angegeben und in erster Linie Trityl darstellen.

Ein durch $Y_7$ und $Y_8$ zusammen gebildeter, abspaltbarer Rest $Y_7 + Y_8$ ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl oder Niederalkoxy sein können, welcher insbesondere Benzyliden, oder Cycloalkyliden, z.B. Cyclopentyliden oder Cyclohexyliden, ferner die Carbonylgruppe darstellt.

Hydrogenolytisch abspaltbare Reste $Y_7$ bzw. $Y_8$ insbesondere gegebenenfalls substituierte 1-Phenylniederalkylgruppen, in erster Linie Benzyl, ferner auch geeignete Acylgruppen, wie gegebenenfalls substituiertes 1-Phenylniederalkoxycarbonyl, sowie durch die Gruppen $Y_7$ und $Y_8$ gemeinsam gebildete, gegebenenfalls substituierte 1-Phenylniederalkylidengruppen können durch Behandeln mit kataly tisch aktiviertem Wasserstoff, z.B. mit Wasserstoff in Gegenwart eines Katalysators, wie eines geeigneten Edelmetallkatalysators, z.B. Palladium oder Platin abgespalten werden.

Hydrolytisch abspaltbare Gruppen $Y_8$ wie Acylreste von organischen Carbonsäuren, z.B. Niederalkanoyl, und von Halbestern der Kohlensäure, z.B. Niederalkoxycarbonyl, ferner z.B. Tritylreste, sowie durch die Reste $Y_7$ und $Y_8$ gemeinsam gebildete Niederalkyliden-, 1-Phenylniederalkyliden- oder Cycloalkylidengruppen sowie Carbonyl $Y_7 + Y_8$ können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $Y_8$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch ein tert.-Niederalkylrest; solche Reste können z.B. durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $Y_8$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl

oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

In der Form von Salzen verwendbare Ausgangsstoffe werden in erster Linie in der Form von Säureadditionssalzen, z.B. mit Mineralsäuren, sowie mit organischen Säuren verwendet.

Bevorzugt sind solche Verbindungen der Formel V, worin $Ac_o$ für Ac steht und $Ac^1_o$ eine Gruppe der Formel

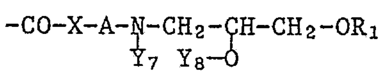

(VI') darstellt, wobei $Y_7$ einen durch H ersetzbaren Substituenten, insbesondere 1-Phenyl-niederalkyl, in erster Linie Benzyl, bedeutet und $Y_8$ Wasserstoff ist. Vorteilhaft wird ein derartiger Substituent $Y_7$ durch Hydrogenolyse durch Wasserstoff ersetzt.

Die Ausgangsstoffe der Formel VI werden beispielsweise erhalten, indem man

ca) eine Verbindung der Formel

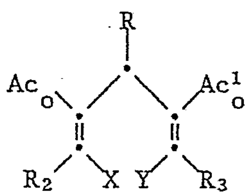

(VIa)

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

cb) eine Verbindung der Formel R-CHO (III) oder ein reaktionsfähiges funktionelles Derivat davon mit einer Verbindung der Formel

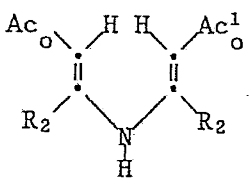

(VIb)

oder einen Tautomeren davon umsetzt oder

cc) eine Verbindung der Formel

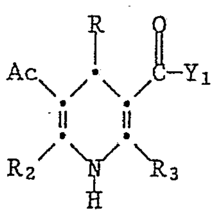

(VII)

mit einer Verbindung der Formel

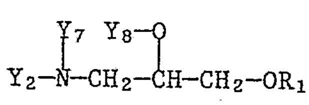

(VIc)

umsetzt, worin $Y_1$ eine Gruppe Y und $Y_2$ eine Gruppe der Formel H-X-A-oder $Y_1$ eine Gruppe der Formel -X-A-Y und $Y_2$ Wasserstoff oder $Y_1$ eine Gruppe der Formel -X-H und $Y_2$ eine Gruppe der Formel Y-A-bedeutet und Y jeweils einen abspaltbaren Rest darstellt, oder

cd) eine Verbindung der Formel

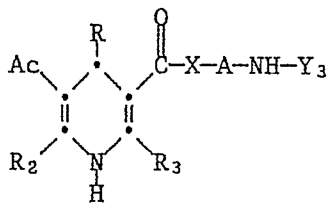

(VII)

mit einer Verbindung der Formel
$Y_4$-$OR_1$ (X)
umsetzt, worin einer der Reste $Y_3$ und $Y_4$ Wasserstoff und der andere eine Gruppe der Formel

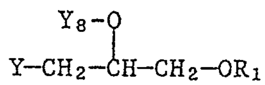

(VIe)

bedeutet, in der Y für einen abspaltbaren Rest steht. Dabei verfährt man beispielsweise wie für die Verfahrensvarianten a), b), d) bzw. e) angegebenen Weise.

Abspaltbare Reste Y gemäss der Verfahrensvariante d) sind beispielsweise Halogenatome, wie Chlor, Brom oder Jod, in Verbindungen VII, worin $Y_1$ für einen abspaltbaren Rest Y steht, ebenso gegebenenfalls veräthertes Hydroxy oder für die Umsetzung mit Verbindungen VII, worin $Y_2$ eine Gruppe H-X-A- und X Imino bedeutet, Aminogruppen, bzw. in Verbindungen VII, worin $Y_1$ eine Gruppe -X-A-Y bedeutet, bzw. in Verbindungen VII, worin $Y_2$ eine Gruppe -A-Y bedeutet, ebenso mit einer Sulfonsäure verestertes Hydroxy. Veräthertes Hydroxy ist beispielsweise Niederalkoxy, z.B. Methoxy, kann aber gegebenenfalls substituiertes Phenoxy, z.B. Phenoxy, p-Nitrophenoxy oder 2,4-Dinitrophenoxy sein. Mit einer Sulfonsäure verestertes Hydroxy ist beispielsweise Niederalkansulfonyloxy, z.B. Methansulfonyloxy, oder gegebenenfalls substituiertes Benzolsulfonyloxy, z.B. Benzol-, p-Toluol- oder p-Brombenzolsulfonyloxy.

Die Umsetzung von Verbindungen VII und VIII gemäss Verfahrensvariante d) erfolgt in üblicher Weise, erforderlichenfalls in Gegenwart eines Kondensationsmittels. Als solche kommen ausgehend von Verbindungen VII und VIII, worin $Y_1$ Halogen oder veräthertes Hydroxy und $Y_2$ eine Gruppe -A-X-H oder $Y_1$ eine Gruppe -X-A-Y und $Y_2$ Wasserstoff oder $Y_1$ eine Gruppe -X-H und $Y_2$ eine Gruppe -A-Y bedeutet, beispielsweise basische Kondensationsmittel, wie Carbonate, Hydroxyde, Alkoholate oder Amide von Alkalimetall- oder Erdalkalimetallen, z.B. Natriumhydroxyd, Kaliumcarbonat, Natriumhydrid, Natriummethanolat, bzw. ausgehend von Verbindungen VII, worin $Y_1$ gegebenenfalls veräthertes Hydroxy bedeutet, saure Mittel, wie Mineralsäuren, beispielsweise Halogenwasserstoffsäuren, z.B. Chlor- oder Bromwasserstoffsäure, oder Schwefelsäure in Betracht. Man kann aber auch die Verbindung VII, worin $Y_1$ eine Gruppe -X-H (X = Oxy) bedeutet, als Carboxylatsalz, z.B. Alkalimetallsalz bzw. eine Verbindung VIII, worin $Y_2$ eine Gruppe -A-X-H (X - Oxy) bedeutet, als Alkoholat, z.B. Alkalimetallalkoholat, bzw. eine Verbindung VII, worin $Y_1$ eine Gruppe -X-H (X = Imino) bedeutet, bzw. eine Verbindung VIII, worin $Y_2$ Wasserstoff oder eine Gruppe -A-X-H (X = Imino) bedeutet, auch als Alkalimetall- oder Erdalkaliamid einsetzen.

Ausgangsstoffe VII, worin $Y_1$ eine Gruppe Y, insbesondere veräthertes Hydroxy, oder -XH, d.h. Hydroxy, Amino oder Niederalkylamino, bedeutet, sind fast sämtlich bekannt. Neue Verbindungen VII werden beispielsweise erhalten, indem man

da) eine Verbindung der Formel

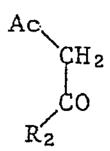

(IIa)

mit einer Verbindung der Formel

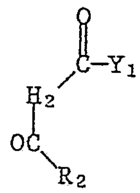

(VIIa)

und Ammoniak oder einem Ammoniak abgebenden Mittel oder

db) eine Verbindung der Formel R-CH=O (III) oder ein reaktionsfähiges funktionelles Derivat davon mit einer Verbindung der Formel

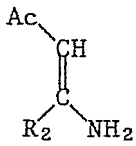

(IIc)

und einer Verbindung IIb oder

dc) eine Verbindung III oder ein reaktionsfähiges Derivat davon mit einer Verbindung der Formel

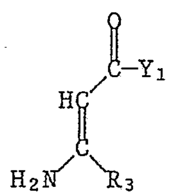

(VIIb)

und einer Verbindung der Formel IIa oder IIc umsetzt.

Verbindungen VII, worin $Y_1$ Halogen, wie Chlor, bedeutet, erhält man beispielsweise

dd) durch Umsetzung einer entsprechenden Carbonsäure (VII; $Y_1$ = OH) z.B. mit Thionylchlorid.

Verbindungen VII, worin $Y_1$ eine Gruppe -X-A-Y darstellt, werden beispielsweise

de) durch Umsetzung einer entsprechenden Carbonsäure (VII; $Y_1$ = OH) bzw. einem entsprechenden Carbonsäureester oder -halogenid (VII; $Y_1$ = veräthertes Hydroxy, Halogen) mit einer Verbindung der Formel H-X-A-Y (VIIc) bzw. mit einer Verbindung der Formel H-X-A-OH (VIId) und anschliessende Ueberführung der Hydroxygruppe in eine Gruppe Y.

In Resten $Y_3$ bzw. $Y_4$ der in Ausgangsstoffen IX bzw. X gemäss Verfahrensvariante e) sind abspaltbare Reste $Y_6$ beispielsweise reaktionsfähige veresterte Hydroxygruppen, wie mit einer Mineralsäure, beispielsweise mit einer Halogenwasserstoffsäure, oder mit einer Sulfonsäure, beispielsweise mit einer Alkansulfonsäure einer Halogensulfonsäure oder einer gegebenenfalls substituierten Benzolsulfonsäure veresterte Hydroxygruppen, z.B. Chlor, Brom oder Jod, Methan- oder Aethansulfonyloxy, Fluorsulfonyloxy, Benzol-, p-Brombenzol- oder p-Toluolsulfonyloxy.

Die Umsetzung von Verbindungen IX und X erfolgt in an sich bekannter Weise, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B., eines Alkalimetall- oder Erdalkalimetallcarbonats oder -hydroxids, oder eines organischen basischen Mittels, wie eines Alkalimetallniederalkanolats, und/oder eines Ueberschusses des basischen Reaktionsteilnehmers und üblicherweise in Gegenwart eines Lösungsmittels oder Lösungsmittelgemisches und, wenn notwendig, unter Kühlen oder Erwärmen, z.B. in einem Temperaturbereich von etwa -20°C bis etwa +150°C, in einem offenen oder geschlossenen Gefäss und/oder in einer Inertgasatmosphäre, z.B. in einer Stickstoffatmosphäre, arbeitet.

Ausgangsstoffe der Formel IX werden beispielsweise hergestellt, indem man eine Verbinding der Formel

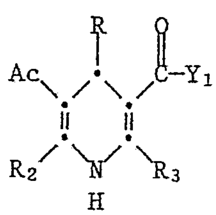

(VII),

worin $Y_1$ gegebenenfalls veräthertes oder verestertes Hydroxy, z.B. Hydroxy, Niederalkoxy oder Halogen, darstellt mit einer Verbindung der Formel $H-X-A-NH_2$ (IXa) und erforderlichenfalls anschliessend mit einer Verbindung der Formel

$Y-CH_2-CH(Y_5)-CH_2-Y_6$ (IXb), worin Y insbesondere Halogen, wie Chlor oder Brom bedeutet, z.B. mit Epichlor- oder Epibromhydrin umsetzt. In analoger Weise erhält man durch Umsetzung von Verbindung X, worin $Y_4$ Wasserstoff ist, mit einer Verbindung IXb auch Verbindungen X, worin $Y_4$ eine Gruppe der Formel XI bedeutet.

Verfahrensgemäss erhältliche Verbindungen der Formel I können in an sich bekannter Weise in andere Verbindungen der Formel I umgewandelt werden, etwa durch Umwandlung von in Verbindungen der Formel I enthaltenen Substituenten in andere von der Formel I umfasste Substituenten.

So kann man z.B. eine Niederalkoxycarbonylgruppe Ac durch Umesterung zu einer anderen Niederalkoxycarbonylgruppe umestern. Dabei führt man die Reaktion z.B. in einem Niederalkanolüberschuss und/oder einem inerten organischen, vorzugsweise oberhalb des Siedepunktes des freizusetzenden Niederalkanols siedenden Lösungsmittel, vorzugsweise in Gegenwart eines Katalysators, z.B. eines Alkalimetallniederalkanolats, wie Natrium- oder Kalium-methanolat oder -äthano lat, oder eines sauren Mittels, z.B. von Salz- oder Schwefelsäure, in der Wärme und üblicherweise unter Abdestillieren des freigesetzten Alkohols durch.

Ferner kann man Verbindungen der Formel I, in welchen R einen Pyridylrest darstellt, in entsprechende N-Oxide unwandeln. Die Oxidation kann in an sich bekannter Weise durchgeführt werden, z.B. durch Behandeln mit organischen Persäuren, wie Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100°C, in Gegenwart oder Abwesenheit von Niederalkansäuren, z.B. Essigsäure. Dabei muss, insbesondere bei Verwendung von Persäuren, darauf geachtet werden, dass keine Ueberoxidation aufgrund einer zu langen Reaktionsdauer eintreten kann.

Thio kann man z.B. auf übliche Weise zu entsprechendem Sulfinyl bzw. Sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig-, p-Nitroperbenzoe-, m-Chlorperbenzoe- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der V. oder VI. Nebengruppe, z.B. Vanadium-, Molybdän-oder Wolframoxid, zu nennen sind. Die Oxidation wird unter milden Bedingungen, z.B. bei Temperaturen von -50° bis etwa +100°C, durchgeführt. Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation von Thio zu Sulfonyl. Jedoch setzt man hierbei üblicherweise das Oxidationsmittel im Ueberschuss ein.

Enthalten die Verbindungen der Formel (I) ungesättigte Reste, wie Niederalkenyloxy- oder Niederalkinyloxygruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80 bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Je nach Reaktionsbedingungen können Verbindungen der Formel I sowie entsprechender Ausgangsverbindungen freier Form oder in Form von Salzen erhalten werden.

So können erhaltene Säureadditionssalze in an sich bekannter Weise, z.B. durch Behandeln mit einer Base, wie einem Alkalimetallhydroxid, in die freien Verbindungen, oder z.B. durch Behandeln mit geeigneten Säuren oder Derivaten davon in andere Salze umgewandelt werden. Erhaltene freie Verbindungen der Formel I können, z.B. durch Behandeln mit Säuren oder entsprechenden Anionenaustauschern, in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn-und zweckgemäss gegebenenfalls auch die entsprechenden Salze bzw. freien Verbindungen zu verstehen.

Die neuen Verbindungen einschliesslich deren Salze können auch in Form ihrer Hydrate erhalten werden, oder ihre Kristalle können z.B. das zur Kristallisation verwendete Lösungsmittel einschliessen.

Verbindungen der Formel I können, je nach chemischer Struktur, Verfahrensreaktion und/oder Art der Ausgangsstoffe, Stereoisomere bilden. Die vorstehenden Verfahrensvarianten können je nach Wahl des Ausgangsmaterials so geführt werden, dass die jeweilige Synthese enantioselektiv verläuft, d.h. eines der

möglichen Enantiomeren ausschliesslich bzw. überwiegend erhalten werden kann.

So können z.B. je nach Anzahl der asymmetrischen C-Atome beispielsweise reine Enantiomere bzw. Diastereomere oder Gemische derselben, wie Enantiomeren- oder Diastereomerengemische vorliegen. In diesem Zusammenhang sei insbesondere auf das mit der Gruppe R verbundene C-Atom des 1,4-Dihydropyridinringes, und auf das mit der in Formel I eingezeichnete Hydroxygruppe verbundenen C-Atome hingewiesen, die unabhängig voneinander R- oder S-Konfiguration aufweist können. Als bevorzugt werden Verbindungen der Formel I angesehen, in denen das mit dem Rest R verbundene C-Atom, sofern Ac Niederalkoxycarbonyl oder Niederalkanoyl darstellt R-Konfiguration bzw., sofern Ac Niederalkansulfonyl darstellt, S-Konfiguration aufweist. Das mit der Hydroxygruppe verbundene C-Atom besitzt vorzugsweise S-Konfiguration.

Erhaltene Diastereomerengemische können auf Grund der physikalisch-chemischen Unterschiede der Racemate in bekannter Weise in die reinen Diastereomeren aufgetrennt werden, beispielsweise durch Chromatographie und/oder fraktionierte Kristallisation.

Enantiomerengemische lassen sich nach an sich bekannten Methoden in die Enantiomeren (optischen Antipoden) zerlegen, beispielsweise durch Umkristallisation aus einem optisch aktiven Lösungsmittel, Chromatographie an chiralen Absorbentien, durch Spalten mit spezifischen, immobilisierbaren Enzymen, über Bildung von Einschlussverbindungen, z.B. unter Verwendung chiraler Kronenäther, wobei nur ein Enantiomeres komplexiert wird, mit Hilfe von geeigneten Mikroorganismen oder durch Umsetzen einer Verbindung der Formel I mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Man kann aber auch von Ausgangsstoffen, z.B. II, IV, V, VII und VIII bzw. IX und X, oder deren Vorstufen mit der gewünschten Konfiguration ausgehen und so zu Zielverbindungen der gewünschten Stereotaxie gelangen oder sich asymmetrischer Synthesemethoden bedienen.

Ferner kann man z.B. Verbindungen der Formel I, worin Ac Niederalkoxycarbonyl ist, unter Verwendung eines optisch aktiven Alkohols nach dem oben beschriebenen Verfahren umestern und das so erhältliche Diastereomerengemisch, z.B. mittels fraktionierter Kristallisation, in die Diastereomeren auftrennen und das gewünschte Enantiomere durch Umesterung einem der gewünschten Niederalkoxycarbonylgruppe entsprechenden Niederalkanol freisetzen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivats, z.B. Salzes, und/oder seiner Racemate bzw. Antipoden verwendet oder unter den Reaktionsbedingungen bildet. Salze von Ausgangsstoffen mit salzbildenden basischen Eigenschaften sind z.B. solche mit Mineralsäuren, wie Salzsäure, Schwefelsäure oder Phosphorsäure, oder mit organischen Säuren, z.B. Essigsäure.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Zwischenprodukte sowie Verfahren zur deren Herstellung bilden ebenfalls einen Gegenstand der vorliegenden Erfindung.

In diesem Zusammenhang sei insbesondere auf Verbindungen der Formel IX, worin $Y_3$ Wasserstoff ist, d.h. auf Verbindungen der Formel

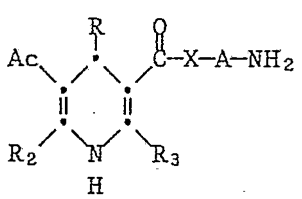

(IXc)

hingewiesen. Diese und ihre pharmazeutisch verwendbaren Salze besitzen wertvolle pharmakologische Eigenschaften, vor allem im cardiovasculären Bereich. Sie zeigen insbesondere antihypertensive Wirkungen, die sich beispielsweise an der renalhypertonischen Ratte in Anlehnung an die von Goldblatt et al. (J. Exptl. Med. 59, 347 (1934) beschriebene Testanordnung in Dosen von etwa oberhalb 30 mg/kg bis oberhalb 100 mg/kg p.o. nachweisen lässt.

So bewirkt z.B. die orale Verabreichung von 100 mg/kg 1,4-Dihydro-2,6-dimethyl-4-(3-nitrophenyl)-pyridin-3,5-dicarbonsäure-3-{2-[p-(2-aminoäthoxy)phenoxy]äthyl}ester-5-methylester an der renalhypertonischen Ratte eine Blutdrucksenkung von ca. 52 mm Hg. Die neuen Verbindungen wirken ferner als Calcium-Antagonisten, wie sich z.B. in in vitro-Versuchen als Hemmung der Calcium-induzierten Vasokonstriktion am isoliert perfundierten Mesenterialbett der Ratte, beispielsweise unter Verwendung von 1,4-Dihydro-2,6-dimethyl-4-(m-nitrophenyl)pyridin-3,5-dicarbonsäure-3-{2-[p-aminoäthoxy)phenoxy]äthyl}ester-5-methylester in einer Konzentration von etwa 0,2 n mol/Liter zeigen lässt.

Ferner lässt sich die Bindung der neuen Verbindungen an Dihydropyridin-Rezeptoren mittels Verdrängung

der Bindung von $^3$H-Nitrendipin an Membranen von Meerschweinchenherz in in vitro-Versuchen nachweisen, etwa in einer Konzentration von etwa 0,2 n Mol/Liter unter Verwendung von 1,4-Dihydro-4-(3-nitrophenyl)-2,6-dimethylpyridin-3,5-dicarbonsäure-3-{2-[p-(2-aminoäthoxy)phenoxy]äthyl}ester-5-methylester.

Die Verbindungen der Formel IXc können dementsprechend nicht nur als Zwischenprodukte zur Herstellung anderer pharmakologisch wirksamer Verbindungen, z.B. solcher der Formel I, sondern auch als Cardiotonika, wie Coronardilatatoren und Antihypertensiva zur Behandlung von cardiovasculären Krankheitszuständen, wie Angina pectoris und ihre Folgen, Gefässspasmen, hohem Blutdruck und Herzinsuffizienz Verwendung bzw. zur Herstellung von Antianginosa, Vasodilatatorika, Antihypertonika und/oder Inotropika finden.

Die Erfindung betrifft somit ebenso die Verwendung von Verbindungen der Formel IXc, worin Ac Niederalkoxycarbonyl, Niederalkanoyl oder Niederalkansulfonyl bedeutet, X Oxy, Imino oder Niederalkylimino darstellt, A einen durch eine Gruppe der Formel $-X_1-Ph-X_2$, worin mindestens einer der Reste $X_1$ und $X_2$ Oxy und ein davon verschiedener Rest $X_1$ oder $X_2$ eine direkte Bindung darstellt und Ph einen unsubstituierten oder durch Niederalkyl, Niederalkoxy, Halogen, Trifluormethyl und/oder Nitro substituierten Phenylenrest bedeutet, unterbrochenen Alkylenrest darstellt, R einen gegebenenfalls durch Niederalkyl, Niederalkoxy, Halogenniederalkoxy, Niederalkenyloxy, Halogen, Trifluormethyl, Cyano, einen gegebenenfalls durch Niederalkyl, Niederalkoxy, Halogen, Trifluormethyl und/oder Nitro substituierten Benzylthio- bzw. Benzyloxyrest und/oder Nitro substituierten Phenylrest, einen gegebenenfalls durch Niederalkyl, Niederalkoxy, gegebenenfalls S-oxidiertes Niederalkylthio und/oder Halogen substituierten, gegebenenfalls 1-oxidierten Pyridylrest oder einen Benzofurazanylrest bedeutet, $R_2$ für Niederalkyl steht und $R_3$ Niederalkyl, Hydroxyniederalkyl, Cyano oder Amino bedeutet, und ihrer Säureadditionssalze als Zwischenprodukte bzw. Arzneimittelwirkstoffe, sowie die vorstehenden Verbindungen selbst, soweit neu, und Verfahren zu ihrer Herstellung.

Dabei haben Ac, X, A, R, $R_2$ und $R_3$ beispielsweise die unter der Formel I, insbesondere für die eingangs als bevorzugt Verbindungsgruppen der Formel I angegebenen Bedeutungen.

Die Erfindung betrifft ebenfalls die Verwendung der Verbindungen der Formel I bzw. IXc oder von pharmazeutisch verwendbaren Salzen von solchen Verbindungen mit salzbildenden Eigenschaften, insbesondere als pharmakologisch wirksame Verbindungen, in erster Linie als Coronardilatatoren und Antihypertensiva zur Behandlung von cardiovasculären Krankheitszuständen, wie Angina pectoris und ihre Folgen, Gefässspasmen, hohem Blutdruckund Herzinsuffizienz. Dabei kann man sie, vorzugsweise in Form von pharmazeutischen Präparaten, in einem Verfahren zur prophylaktischen und/oder therapeutischen Behandlung des tierischen und menschlichen Körpers, insbesondere zur Behandlung von cardiovasculären Krankheitszuständen, wie Angina pectoris und ihre Folgen, Gefässspasmen, hohem Blutdruck und Herzinsuffizienz verwenden.

Die Dosierung des Wirkstoffs, der allein oder zusammen mit dem üblichen Träger- und Hilfsmaterial verabreicht wird, hängt von der zu behandelnden Spezies, deren Alter und individuellen Zustand, sowie der Verabreichungsweise ab. Die täglichen Dosen liegen z.B. für Mammalien mit einem Körpergewicht von etwa 70 kg, je nach Art der Erkrankung, individuellem Zustand und Alter, vorzugsweise zwischen etwa 100 und 1000 mg, insbesondere zwischen etwa 150 mg und etwa 500 mg, und speziell etwa 150 mg bis 250 mg, jeweils bei oraler Verabreichung.

Die Erfindung betrifft im weiteren pharmazeutische Präparate, die Verbindungen der Formel I bzw. IXc oder pharmazeutisch verwendbare Salze von solchen Verbindungen mit salzbildenden Eigenschaften als Wirkstoffe enthalten, Verfahren zu ihrer Herstellung, sowie die Verwendung von Verbindungen der Formel I oder deren pharmazeutisch verwendbaren Salze zur Herstellung von Arzneimitteln als Coronardilatoren und Antihypertensiva zur Behandlung von cardiovasculären Krankheitszuständen, wie Angina pectoris und ihre Folgen, Gefässspasmen, zentrale und periphere Durchblutungsstörungen, hohem Blutdrück, Arrhythmien und Herzinsuffizienz, ferner zur Anwendung als Hemmer der Plättchenaggregation.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwa 300 mg, insbesondere von etwa 20 mg bis etwa 200 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride oder Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die folgenden Beispiele dienen zur Illustration der Erfindung; Temperaturen werden in Celsiusgraden angegeben.

Beispiel 1: Ein Gemisch von 4,1 g (8,3 mMol) 1,4-Dihydro-2,6-dimethyl-4-(m-nitrophenyl)-pyridin-3,5-dicarbonsäure-3-{2-[2-aminoäthyl)phenoxy]äthyl}ester-5-methylester und 1,6 g 2-(2,3-Epoxypropoxy)benzonitril in 20 ml Isopropanol wird 2 Stunden zum Rückfluss erhitzt und dann unter vermindertem Druck zur Trockne eingedampft. Der Eindampfrückstand wird in 20 ml 1n-Methansulfonsäure gelöst und mit 40 ml Essigsäureäthylester extrahiert. Die Wasserphase wird abgetrennt und mit 2n-Natronlauge alkalisch gestellt. Das durch Extraktion mit Aethylacetat erhaltene Rohprodukt wird über Kieselgel "flash"-chromatographiert unter Verwendung von Toluol/Isopropanol/-konzentriertem Ammoniak (170:30:3) als Laufmittel. Man erhält so 1,4-Dihydro-2,6-dimethyl-4-(m-nitrophenyl)-pyridin-3,5-dicarbonsäure-3-{{{2-{{p-{2-[3-(o-cyanophenoxy)-2-hydroxypropylamino]äthyl}phenoxy}}äthyl}}}ester-5-methylester als amorphen Schaum.

$^1$H-NMR ($CDCl_3$): 2,3 (6,2 s); 2,5-3,0 (6,m); 3,0-3,1 (3,m); 3,63 (3,s); 4,0-4,2 (4,m); 4,2-4,4 (2,m); 4,4-4,5 (1,m); 5,1 (1,s); 6,0 (1,s); 6,75 (2,d,9Hz); 7,0 (2,m); 7,15 (2,d,9Hz); 7,3 (1,5,7Hz); 7,5-7,55 (2,m); 7,63 (1,d,7Hz); 7,9 (1,2d,2Hz,7Hz); 8,05 (1,m).

Der als Ausgangsmaterial benötigte Ester kann auf folgende Weise hergestellt werden:

1a) 24,2 g (177 mM) Tyramin werden in 180 ml Dioxan mit 39,0 g Pyrokohlensäureditertiärbutylester umgesetzt. Nach 2 bis 3 Stunden wird das Dioxan im Vakuum abgedampft. Der verbleibende Rückstand rohes N-Tertiärbutyloxycarbonyltyramin bildet ein allmählich kristallin erstarrendes Oel, das roh in die folgende Reaktion eingesetzt werden kann.

1b) Ein Gemisch von 8,6 g (39 mM) rohem N-Tertiärbutoxycarbonyltyramin, 27,6 g (200 mM) Kaliumcarbonat und 73,3 g (390 mM) 1,2-Dibromäthan werden unter Rühren 4 Stunden zum Siedem erhitzt. Das Reaktionsgemisch wird abgekühlt, mit 200 ml Wasser und 400 ml Essigsäureäthylester versetzt und die organische Phase abgetrennt. Nach dem Eindampfen unter vermindertem Druck verbleibt ein kristalliner Rückstand, der nach Umkristallisation aus 2-Propanol N-Tertiärbutyloxycarbonyl-2-[p-(2-bromäthoxy)phenyl]äthylamin vom Smp. 70 - 72° ergibt.

1c) 3,3 g (10 mM) 1,4-Dihydro-2,6-dimethyl-4-(m-nitrophenyl)-pyridin-3,5-dicarbonsäure-3-methylester, 3,5 g (10 mM) der Bromäthoxy-Verbindung aus Stufe 1b) und 2,8 g (20 mM) Kaliumcarbonat werden in 50 ml Acetonitril under Rühren und Rückfluss zum Sieden erhitzt. Nach etwa 20 Stunden wird das Reaktionsgemisch filtriert und das Filtrat unter vermindertem Druck eingedampft. Der Eindampfrückstand wird in Essigsäureäthylester gelöst, mit Wasser gewaschen und die organische Phase im Vakuum eingedampft. Man erhält so rohen 1,4-Dihydro-2,6-dimethyl-4-(m-nitrophenyl)-pyridin-3,5-dicarbonsäure-3-{-[p-(2-tertiärbutyloxycarbonylaminoäthyl)phenoxy]äthyl}ester-5-methylester, der roh weiter verwendet wird.

1d) 5,95 (10 mM) des in Stufe c) beschriebenen Tertiärbutyloxycarbonyl-Derivates werden in 30 ml Ameisensäure gelöst, die Lösung wird bei 20 - 25° während 15 Stunden stehen gelassen und hierauf im Vakuum eingedampft. Der Eindampfrückstand wird in 100 ml 0,1-n. HCl gelöst, die Lösung mit 100 ml

Toluol extrahiert und wässerige Phase mit 2-n. Natronlauge alkalisch gestellt. Durch Extraktion mit Aethylacetat und Eindampfen der Lösung erhält man 1,4-Dihydro-2,6-dimethyl-4-(m-nitrophenyl)-pyridin-3,5-dicarbonsäure-3-{2-[p-(2-aminoäthyl)phenoxy]äthyl}ester-5-methylester als gelben Schaum. Die Verbindung bildet ein Hydrochlorid vom Smp. 196 - 198° (aus Isopropanol).

Beispiel 2: Eine Lösung von 3,5 g (6,8 mM) 1,4-Dihydro-2,6-dimethyl-4-(m-nitrophenyl)-pyridin-3,5-dicarbonsäure-3-{2-[p-(2-aminoäthoxy)phenoxy]äthyl}ester-5-methylester und 1,55 g (8,9 mM) 2-(2,3-Epoxypropoxy)benzonitril in 20 ml Isopropanol wird 2 Stunden zum Rückfluss erhitzt. Das nach dem Eindampfen erhaltene Oel (etwa 6 g) wird durch "flash"-Chromatographie analog Beispiel 1 gereinigt und ergibt 1,4-Diydro-2,6-dimethyl-4-(m-nitrophenyl)-pyridin-3,5-dicarbonsäure-3-{{{2-{{p-{2-[3-(o-cyanophenoxy)-2-hydroxypropylamino]äthoxy}phenoxy}}äthyl}}}ester-5-methylester als amorphen Festkörper.
$^1$H-NMR ($CDCl_3$): 2,3 (6,2s); 3,0-3,1 (4,m); 3,6 (3,s); 4,05 (4,m); 4,14 (3,s); 4,3-4,5 (2,m); 5,1 (1,s); 5,85 (1,s); 6,76 (2,d,10Hz); 6,83 (2,d,10Hz); 7,0 (2,t,8Hz); 7,2-7,3 (1,m); 7,5 (2,m); 7,6 (1,2d,10Hz,2Hz); 7,9 (1,m); 8,05 (1,m).

Das Ausgangsmaterial kann auf folgende Weise hergestellt werden:

2a) 38,4 g (250 mM) 4-(2-Aminoäthoxy)phenol wird mit 54,7 g (250 mM) Pyrokohlensäureditertiärbutylester in 500 ml Dioxan 2 Stunden bei Raumtemperatur gerührt. Nach Zugabe von 20 ml konzentrierten Ammoniaks wird weitere 30 Minuten gerührt. Nach dem Eindampfen unter vermindertem Druck wird in Essigsäureäthylester gelöst, mit Wasser gewaschen und die organische Phase getrocknet ($MgSO_4$) und eingedampft. Das erhaltene ölige Rohprodukt wird aus Cyclohexan-, Aether (10:1) umkristallisiert und ergibt p-(2-Tertiärbutyloxycarbonylaminoäthoxy)phenol vom Smp. 84 - 85°.

2b) 45,5 g (180 mM) des unter 2a) erhaltenen Phenol-Derivates, 340 g (1,8 M) 1,2-Dibromäthan und 124 g (0,9 M) Kaliumcarbonat werden unter Rühren und Rückfluss 7 Stunden zum Sieden erhitzt. Nach dem Abkühlen wird filtriert, das Filtrat mit 1 l Essigsäureäthylester verdünnt, mit 300 ml Wasser gewaschen, über Magnesiumsulfat getrocknet und unter vermindertem Druck zur Trockne eingedampft. Das kristalline Rohprodukt wird aus Isopropanol umkristallisiert und ergibt 1-(2-Bromäthoxy)-4-(2-tertiärbutyloxycarbonylaminoäthoxy)benzol vom Smp. 84 - 85°.

2c) 8,7 g (24 mM) des in Stufe 2b) erhaltenen Brom-Derivates, 8,0 g (24 mM) 1,4-Dihydro-2,6-dimethyl-4-(m-nitrophenyl)-3,5-pyridindicarbonsäure-monomethylester und 6,6 g (48 mM) Kaliumcarbonat werden 20 Stunden unter Rühren und Rückfluss in 100 ml Acetonitril zum Sieden erhitzt. Aufarbeitung analog Beispiel 1c ergibt 1,4-Dihydro-2,6-dimethy-4-(m-nitrophenyl)-pyridin-3,5-dicarbonsäure-3-{2-[p-(2-tertiärbutyloxycarbonylaminoäthoxy)phenoxy]äthyl}ester-5-methylester, das ohne weitere Reinigung in die folgende Stufe eingesetzt wird.

2d) 14,6 g des in der Stufe 2c) erhaltenen Produktes werden in 50 ml Ameisensäure gelöst, die Lösung wird 6 Stunden bei Raumtemperatur stehen gelassen und analog Beispiel 1d) aufgearbeitet. Man erhält so ein kristallines Rohprodukt, das nach Umkristallisation aus Aceto nitril 1,4-Dihydro-2,6-dimethyl-4-(m-nitrophenyl)-pyridin-3,5-dicarbonsäure-3-{2-[p-(2-aminoäthoxy)phenoxy]äthyl}ester-5-methylester vom Smp. 153-154° ergibt.

Beispiel 3: Eine Lösung von 5,4 g (10 mMol) 1,4-Dihydro-2,6-dimethyl-4-(m-nitrophenyl)-pyridin-3,5-dicarbonsäure-3-{2-[p-(2-amino-2-methyl-propyloxy)phenoxy]äthyl}ester-5-methylester und 2,3 g (13 mMol) 2-(2,3-Epoxypropoxy)benzonitril in 30 ml Isopropanol werden 18 Stunden unter Rückfluss erhitzt. Nach dem Aufarbeiten und Chromatographieren analog Beispiel 1 erhält man 1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]2-methyl-propyloxy}-phenoxy}}äthyl}}}ester-5-methylester als amorphen Festkörper.
$^1$H-NMR (DMSO-$d_6$): 1,1 (6,s); 1,9 (1,b); 2,3 (6, 2s); 2,6-2,8 (2, m); 3,52 (3, s); 3,67 (2, s); 3,85 (1, b); 4,0 - 4,1 (4, m); 4,1 - 4,2 (2, b); 5,0 (1, s); 5,1 (1, b); 6,80 (2, d, 8Hz); 6,82 (2, d, 8Hz); 7,07 (1, t, 7Hz); 7,24 (1, d, 8Hz); 7,44 (1, t, 7Hz); 7,6 - 7,75 (3, m); 7,94 (1, s); 8,0 (1,m); 10,6 (1, s).

Das Ausgangsmaterial kann auf folgende Weise hergestellt werden:

3a) Ein Gemisch von 100,0 g (0,5 Mol) Hydrochinonmonobenzyläther, 124 g (0,75 Mol) Methansulfonsäure-2-methyl-2-nitro-propylester, 207 g Kaliumcarbonat und 500 ml Dimethylformamid wird während 70 - 80 Stunden in einem Bad von 130 - 140° gerührt. Nach dem Abkühlen wird filtriert und das Filtrat im Vakuum eingedampft. Das verbleibende schwarze Oel wird in Aethylacetat (ca. 1l) gelöst, die Lösung mit 200 ml 2-n Natronlauge extrahiert, mit Wasser gewaschen, getrocknet ($MgSO_4$) und eingedampft. Nach Chromatographie über 1 kg Kieselgel (Elution mit Aethylacetat) erhält man rohen Hydrochinon-1-benzyl-4-(2-methyl-2-nitro-propyl)äther, der nach Umkristallisation aus Methanol bei 75-76° schmilzt.

3b) 26 g (86 mMol) Hydrochinon-1-benzyl-4-(2-methyl-2-nitro-propyl)äther werden in 400 ml Methanol über 2,6 Pd/C (5 o/o) bei 50 bar und 50 - 55° hydriert bis 4 Aequivalent Wasserstoff aufgenommen wurden (ca. 16 Std.). Nach Filtration und Eindampfen erhält man einen kristallinen Rückstand, der nach Umkristallisation aus Isopropanol Hydrochinon-mono-(2-amino-2-methyl-propyl)äther vom Smp. 165-166° ergibt.

3c) 13,8 g (76 mM) Hydrochinon-mono-(2-amino-2-methyl-propyl)äther und 16,6 g (76 mM) Pyrokohlensäureditertiärbutylester werden analog Beispiel 2a) umgesetzt und aufgearbeitet. Man erhält nach Umkristallisation aus Aether-Cyclohexan p-[2-(Tertiärbutyloxycarbonylamino)-2-methyl-propoxy]phenol vom Smp. 121 - 123°.

3d) Ein Gemisch von 19,2 g (68 mM) p-[2-(Tertiärbutyloxycarbonylamino)-2-methyl-propoxy]phenol, 47 g (340 mM) Kaliumcarbonat und 1,5 g Triäthyl-benzylammoniumchlorid wird in 56 ml (680 mM)

1,2-Dibromäthan analog Beispiel 2b) umgesetzt und aufgearbeitet. Man erhält so rohes 1-(2-Bromäthoxy)-4-[2-(tertiärbutyloxycarbonylamino)-2-methyl-propoxy]benzol als Oel, das roh weiterverwendet wird.

3e) 16,6 g (50 mM) 1,4-Dihydro-2,6-dimethyl-4-(m-nitrophenyl)-3,5-pyridindicarbonsäure-monomethylester, 19,4 g (50 mM) rohes 1-(2-Bromäthoxy)-4-[2-(tertiärbutyloxycarbonylamino)-2-methyl-propoxy]benzol, 13,8 g Kaliumcarbonat und 0,6 g Triäthyl-benzyl-ammoniumchlorid werden in 500 ml Acetonitril 16 Stunden unter Rühren und Rückfluss zum Sieden erhitzt. Das Reaktionsgemisch wird filtriert, eingedampft und in 500 ml Aethylacetat gelöst. Nach Waschen mit 100 ml Wasser und Trocknen ($MgSO_4$) wird das Lösungsmittel abgedampft und der rohe Rückstand ohne weitere Reinigung mit 50 ml Dioxan und 100 ml Ameisensäure versetzt und 7 - 10 Stunden bei Raumtemperatur im Dunkeln stehen gelassen. Nach dem Eindampfen im Vakuum wird der Rückstand in 2n- Salzsäure gelöst und die Lösung mit Toluol extrahiert. Die wässrige Phase wird mit 2n-Natronlauge alkalisch gestellt und mit Aethylacetat extrahiert. Man erhält nach dem Trocknen der Lösung ($MgSO_4$) und Eindampfen ein Oel aus dem aus Acetonitril 1,4-Dihydro-2,6-dimethyl-4-(m-nitrophenyl)-pyridin-3,5-dicarbonsäure -3-{2-[p-(2-amino-2-methyl-propyloxy)phenoxy]äthyl}ester-5-methylester vom Smp. 141 - 142° erhalten werden.

Beispiel 4: 2,7 g (5 mM) 1,4-Dihydro-2,6-dimethyl-4-(m-nitrophenyl)pyridin-3,5-dicarbonsäure-3-{2-[p-(2-amino-2-methyl-propyloxy)phenoxy]äthyl}ester-5-methylester und 1,46 g (6,5 mM) 1-(2,3-Epoxypropoxy)-4-(2-methoxyäthoxy)-benzol in 20 ml Isopropanol werden analog Beispiel 3 umgesetzt und aufgearbeitet. Nach "flash"-Chromatographie an Kieselgel mittels eines Gemisches von Toluol/Isopropanol/konzentriertem Ammoniak (170:30:3) erhält man 1,4-Dihydro-2,6-dimethyl-4-(m-nitrophenyl)-pyridin-3,5-dicarbonsäure-3-{{{2-{{p-{2-[[3-[p-(2-methoxyäthoxy)phenoxy]-2-hydroxy-propylamino]]2-methyl-propyloxy}phenoxy}}äthyl}}}ester-5-methylester als amorphen Schaum.
$^1$H-NMR (DMSO-$d_6$): 1,1 (6, s); 2,28 (6, 2s); 2,5 - 2,7 (2, m); 3,28 (3, s); 3,52 (3, s); 3,58 - 3,68 (4, m); 3,7 - 3,8 (3, m); 4,0 - 4,1 (4, m); 4,25 (2, m); 5,0 (1, s); 5,0 (1, b); 6,80 - 6,85 (4, m); 7,45 (1, t); 7,6 (1, d); 7,94 (2, m); 9,1 (1, s).

Beispiel 5: In analoger Weise wie in den Beispielen 1 bis 4 beschrieben erhält man:
1,4-Dihydro-4-(m-nitrophenyl)-2,6-dimethyl-pyridin-3,5-dicarbonsäure3-{{{2-{{p-{2-[3-(o-cyanophenoxy)-2-hydroxy-propylamino]propyloxy}phenoxy}}äthyl}}}ester-5-methylester;
1,4-Dihydro-4-(m-nitrophenyl)-2,6-dimethyl-pyridin-3,5-dicarbonsäure-3-{{{2-{{o-{2-[3-(o-cyanophenoxy)-2-hydroxy-propylamino]äthoxy}phenoxy}}äthyl}}}ester-5-methylester;
1,4-Dihydro-4-(m-nitrophenyl)-2,6-dimethyl-pyridin-3,5-dicarbonsäure-3-{{{2-{{p-{2-[[3-[p-(2-methoxyäthoxy)phenoxy]-2-hydroxy-propylamino]]äthoxy}phenoxy}}äthyl}}}ester-5-methylester;
1,4-Dihydro-4-(m-nitrophenyl)-2,6-dimethyl-pyridin-3,5-dicarbonsäure3-{{{2-{{p-{2-[[3-[p-(2-isopropyloxyäthoxymethyl__phenoxy]-2-hydroxy-propylamino]]äthoxy}phenoxy}}äthyl}}}ester-5-methylester;
1,4-Dihydro-4-(m-methoxyphenyl)-2,6-dimethyl-pyridin-3,5-dicarbonsäure3-{{{3-{{p-{2-[3-(o-cyanophenoxy)-2-hydroxy-propylamino]äthyl}phenoxy}}propyl}}}-ester-5-methylester und
1,4-Dihydro-4-(m-nitrophenyl)-2,6-dimethyl-pyridin-3,5-dicarbonsäure3-{{{3-{{p-{2-[3-(o-cyanophenoxy)-2-hydroxy-propylamino]äthyl}phenoxy}}propyl}}}ester-5-methylester.

Beispiel 6: Eine Lösung von 4,6 g (8,5 mM) 1,4-Dihydro-2,6-dimethyl-4-(m-nitrophenyl)-pyridin-3,5-dicarbonsäure-3-{2-[p-(2-amino-2-methyl-propyloxy)phenoxy]äthyl}ester-5-methylester und 2,95 g (11 mM) 1-(2,3-Epoxy-propoxy)-4-[2-(isopropoxy)-äthoxy-methyl]-benzol in 20 ml Isopropanol wird 6 Stunden unter Rühren zum Rückfluss erhitzt. Aufarbeitung und chromatographische Reinigung analog Beispiel 1 gibt 1,4-Dihydro-2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propylamino]]-2-methyl-propoxy}phenoxy}}äthyl}}}ester-5-methylester als glasartigen Festkörper der Struktur:

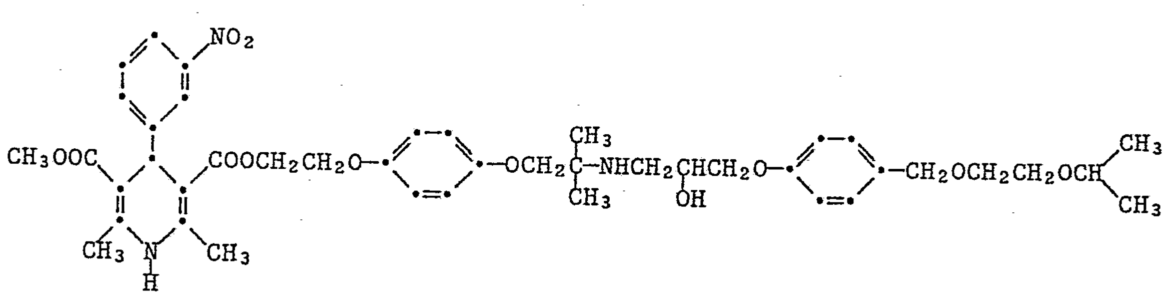

$^1$H-NMR (DMSO-$d_6$): 1,06 - 1,12 (12, 1s + 1d); 2,30 (6, 2s); 2,5 - 2,7 (2, m); 3,48 (3, s); 3,53 (3, s); 3,67 (2, s); 3,7 - 4,3 (7, m); 4,40 (2, s); 5,0 (2, s + b); 6,7 - 8,0 (12 arom. H); 9,1 (1 nH, s).

Beispiel 7: Eine Lösung von 5,11 g (10 mM) 1,4-Dihydro-2,6-dimethyl-4-(m-nitrophenyl)-pyridin-3,5-dicarbonsäure-3-{2-[p-(2-aminoäthoxy)phenoxy]äthyl}ester-5-methylester und 3,2 g (12 mM) 1-(2,3-Epoxypropoxy)-4-[2-(isopropoxy)-äthoxy-methyl]-benzol in 30 ml Isopropanol wird 4 Stunden unter Rühren und Rückfluss zum Sieden erhitzt. Nach Abdampfen des Lösungsmittels wird der EindampfRückstand an 1 kg Kieselgel "flash"-chromatographiert (Toluol, Isopropanol, conc. Ammoniak 170:30:3). Die das gewünschte Produkt enthaltenden Fraktionen werden eingedampft und ergeben 1,4-Dihydro2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propyla-

mino]]äthoxy}-phenoxy}}-äthyl}}}ester-5-methylester als glasartigen Festkörper der Struktur

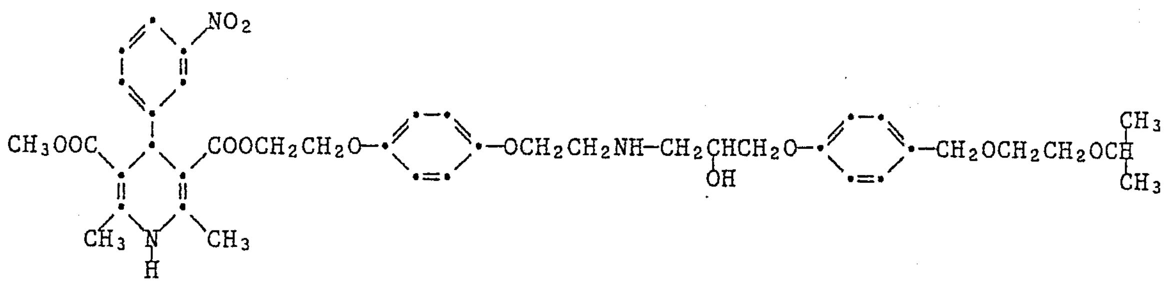

$^1$H-NMR (DMSO-$d_6$): 1,15 (6, d); 2,30 (6, 2s); 2,6 - 2,8 (2, m); 2,90 (2, t); 3,50 (3, s); 3,55 (4, s); 3,50 - 3,58 (1, m); 3,9 - 4,0 (6, m); 4,1 (2, m); 4,3 (2, m); 5,0, (1, s); 5,1 (1, b.); 6,7 - 7,95 (12 arom. H); 9,1 (1, s).

Beispiel 8: Analog Beispiel 7 kann man 1,4-Dihydro-2,6-dimethyl-4-(m-nitrophenyl)-pyridin-3,5-dicarbonsäure-3-{{{2-{{p-{2-[[3-[4-(2-methoxyäthyl)phenoxy]-2-hydroxy-propylamino]]äthoxy}phenoxy}}äthyl}}}ester-5-methylester als dickflüssiges Oel herstellen:

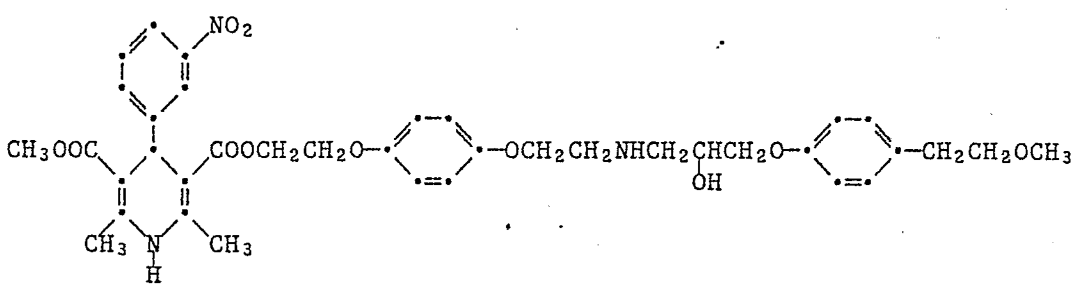

$^1$H-NMR (DMSO-$d_6$): Analog Beispiel 7, abweichende Signale bei 2,72 ppm (2, t) Ar$CH_2$$CH_2$-O; 3,46 (2, t) Ar$CH_2$$CH_2$O-; 3,21 (3, s) -O$CH_3$.

Beispiel 9: Analog Beispiel 7 kann man 1,4-Dihydro-2,6-dimethyl-4-(m-nitrophenyl)-pyridin-3,5-dicarbonsäure-3-{{{2-{{p-{2-[[3-[4-(2-methoxyäthoxy)phenoxy]-2-hydroxy-propylamino]]äthoxy}phenoxy}}äthyl}}}ester-5-methylester als Harz herstellen:

$^1$H-NMR (DMSO-$d_6$): Analog Beispiel 7; abweichende Signale bei 3,28 ppm (3, s) -O$CH_3$; 3,62 (2, t) ArO$CH_2$$CH_2$O-$CH_3$; 3,98 (2, t) ArO$CH_2$$CH_2$O$CH_3$.

Beispiel 10: Analog Beispiel 7 kann man 1,4-Dihydro-2,6-dimethyl-4-(m-nitrophenyl)-pyridin-3,5-dicarbonsäure-3-{{{2-{{p-{2-[[3-[4-(2-cyclopropylmethoxy-äthyl)phenoxy]-2-hydroxy-propylamino]]äthoxy}phenoxy}}äthyl}}}ester-5-methylester als Harz herstellen:

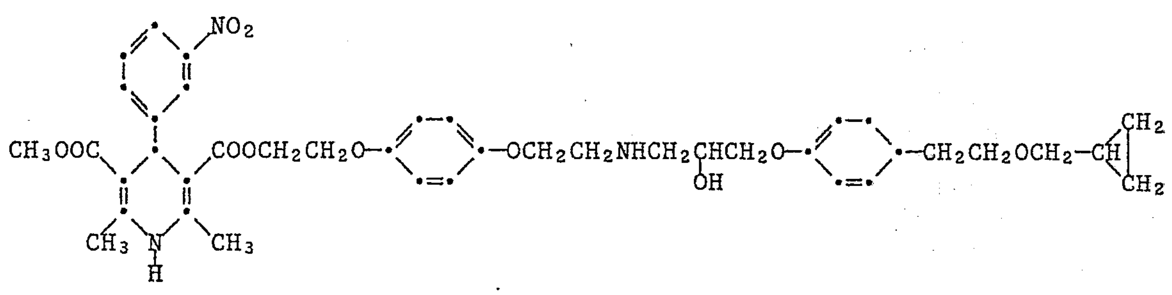

$^1$H-NMR (DMSO-$d_6$): Analog Beispiel 7; abweichende Signale bei 0,13 ppm (2, m) und 0,44 (2, m)

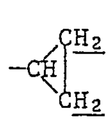 ;

3,22 (2, d).

Beispiel 11: Analog Beispiel 7 kann man 1,4-Dihydro-2,6-dimethyl-4-(m-nitrophenyl)-pyridin-3,5-dicarbonsäure-3-{{{2-{{p-[[3-[4-(2-isopropoxy-äthoxy)phenoxy]-2-hydroxy-propylamino]]äthoxy}phenoxy}}äthyl}}}ester-5-methylester als Harz herstellen:

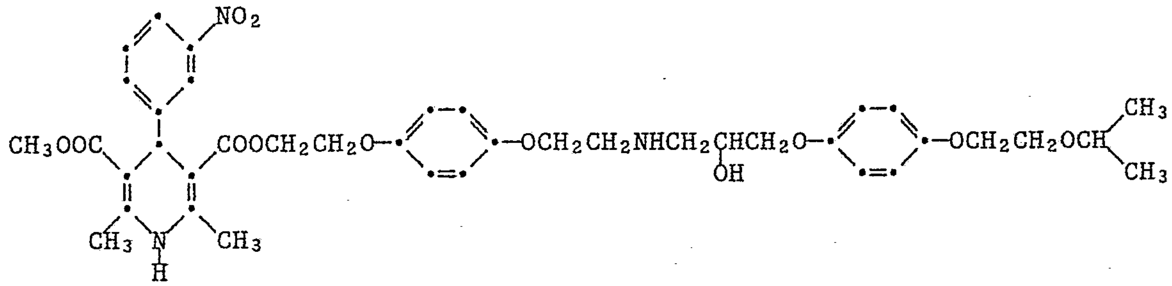

$^1$H-NMR (DMSO-$d_6$): Analog Beispiel 7; abweichende Signale bei 1.08 (6, d) $-CH(\underline{CH_3})_2$; 3,56 - 3,66 (3, m) $-\underline{CH_2}OCH$.

Beispiel 12: Eine Lösung von 3,0 g (5,86 mM) 1,4-Dihydro-2,6-dimethyl-4-(m-nitrophenyl)-pyridin-3,5-dicarbonsäure -3-{2-[o-(2-aminoäthoxy)phenoxy]äthylester-5-methylester und 2,0 g (7,6 mM) 1-(2,3-Epoxy-propoxy)-4-[2-(isopropoxy)-äthoxymethyl]-benzol in 50 ml Isopropanol wird unter Rühren und Rückfluss 2 Stunden zum Sieden erhitzt. Aufarbeitung und chromatographische Reinigung analog Beispiel 7 ergibt 1,4-Dihydro-2,6-dimethyl-4-(m-nitrophenyl)-pyridin-3,5-dicarbonsäure-3-{{{2-{{o-{2-[[3-[4-(2-isopropoxy-äthoxymethyl)-phenoxy]-2-hydroxy-propylamino]]äthoxy}phenoxy}}äthyl}}}ester-5-methylester als harzartigen Festkörper der Struktur

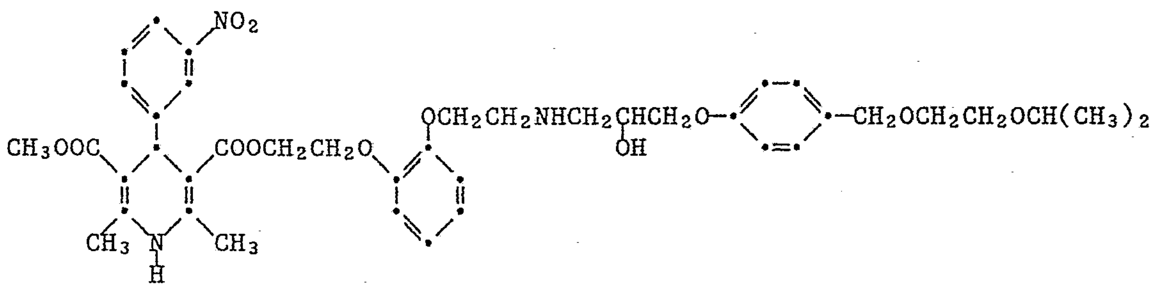

Das Ausgangsmaterial kann auf folgende Weise hergestellt werden:

12a) Analog Beispiel 2a erhält man aus o-(2-Amino-äthoxy)phenol und Pyrokohlensäurediteriärbutylester o-(2-Tertiärbutyloxycarbonyl-amino-äthoxy)phenol als Oel, das kristallin erstarrt und roh weiterverwendet wird.

12b) Umsatz von rohem o-(2-Tertiärbutyloxycarbonyl-amino-äthoxy)phenol mit 1,2-Dibromäthan analog Beispiel 2b, ergibt 1-(2-Bromäthoxy)-2-(2-tertiärbutyloxycarbonylaminoäthoxy)benzol vom Smp. 74-75° (aus Isopropanol).

12c) und 12d) Analog den Stufen 2c) und 2d) erhält man aus der unter 12b, genannten Verbindung den 1,4-Dihydro-2,6-dimethyl-4-(m-nitrophenyl)-pyridin-3,5-dicarbonsäure-3-{2-[o-(2-aminoäthoxy)phenoxy]äthylester-5-methylester nach "flash"-chromatographischer Reinigung (Chloroform-Methanol/8:1) als gelben Schaum.

Beispiel 13: Ein Gemisch von 2,0 g (4,0 mM) 1,4-Dihydro-2,6-dimethyl-4-(m-methoxyphenyl)-pyridin-3,5-dicarbonsäure-3-{2-[p-(2-aminoäthoxy)phenoxy]äthyl}ester-5-methylester und 1,4 g (5,2 mM) 1-(2,3-Epoxypropoxy)-4-[2-isopropoxy)-äthoxymethyl)benzol in 10 ml Isopropanol wird 3 Stunden unter Rühren und Rückfluss zum Sieden erhitzt. Aufarbeitung analog Beispiel 7 ergibt 1,4-Dihydro-2,6-dimethyl-4-(m-methoxyphenyl)-pyridin-3,5-dicarbonsäure-3-{{{2-{{p-{2-[[3-[4-(2-isopropoxy-äthoxymethyl)-phenoxy]-2-hydroxypropylamino]]äthoxy}phenoxy}}-äthyl}}}ester-5-methylester als glasartigen Festkörper der Struktur

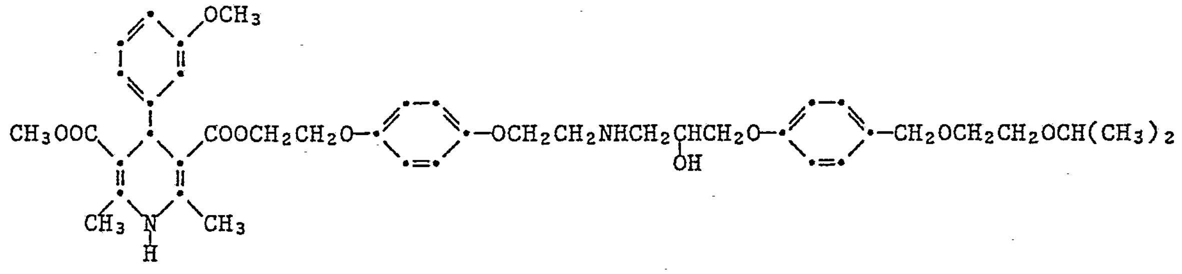

Das Ausgangsmaterial kann auf folgende Weise hergestellt werden:

13a) Aus 3-Methoxybenzaldehyd, 3-Aminocarbonsäuremethylester und Acetessigsäure-(2-cyanoäthylester) erhält man nach bekanntem Verfahren den 1,4-Dihydro-2,6-dimethyl-4-(m-methoxyphenyl)-3,5-dicarbonsäure-monomethylester vom Smp. 198-200° (sintert ab 194°), der als Rohprodukt weiterverwendet wird.

13b) Analog den Beispielen 2c) und 2d) erhält man aus der in 13a) beschriebenen Verbindung den 1,4-Dihydro-2,6-dimethyl-4-(m-methoxyphenyl)-pyridin-3,5-dicarbonsäure-3-{2-[p-(2-aminoäthoxy)phenoxy]äthyl}ester-5-methylester vom Smp. 144-146° (aus Acetonitril).

Beispiel 14: In analoger Weise wie in den Beispielen 1-13 beschrieben kann man herstellen:
1,4-Dihydro-2,6-dimethyl-4-(m-nitrophenyl)-pyridin-3,5-dicarbonsäure-{{{2-{{p-{2-[[3-[4-(2-cyclopropyl-methoxy-äthyl)-phenoxy]2-hydroxy-propylamino]]-2-methyl-propyloxy}phenoxy}}äthyl}}}ester-5-methyl-ester;
1,4-Dihydro-2,6-dimethyl-4-(m-methoxyphenyl)-pyridin-3,5-dicarbonsäure-{{{2-{{p-{2-[[3-[4-(2-cyclopropylmethoxy-äthyl)-phenoxy]-2-hydroxy-propylamino]]-äthoxy}phenoxy}}-äthyl}}}ester-5-methylester;
1,4-Dihydro-2,6-dimethyl-4-(m-nitrophenyl)-pyridin-3,5-dicarbonsäure-{{{2-{{m-{2-[[3-[4-(2-isopropyloxy-äthoxy-methyl)phenoxy]-2-hydroxy-propylamino]]-äthyl}phenoxy}}-äthyl}}}ester-5-methylester;
1,4-Dihydro-2,6-dimethyl-4-(m-methoxyphenyl)-pyridin-3,5-dicarbonsäure-{{{2-{{p-{2-[[3-[4-(2-methoxy-äthoxy)phenoxy]-2-hydroxypropylamino]]-äthoxy}phenoxy}}äthyl}}}ester-5-methylester, Smp. 65-67°;
1,4-Dihydro-2,6-dimethyl-4-(m-methoxyphenyl)-pyridin-3,5-dicarbonsäure-{{{2-{{p-{2-[[3-[4-(2-isopropyloxy-äthoxy)-phenoxy]-2-hydroxy-propylamino]]-äthoxy}phenoxy}}äthyl}}}ester-5-methylester;
1,4-Dihydro-2,6-dimethyl-4-(2,3-dichlorphenyl)-pyridin-3,5-dicarbonsäure-{{{2-{{p-{2-[[3-[4-(2-isopropyloxy-äthoxymethyl)-phenoxy]-2-hydroxy-propylamino]]äthoxy}phenoxy}}äthyl}}}-ester-5-methylester;
1,4-Dihydro-2,6-dimethyl-4-(m-nitrophenyl)-pyridin-3,5-dicarbonsäure-{{{2-{{p-{3-[[3-[4-(2-isopropyloxy-äthoxymethyl)-phenoxy]-2-hydroxy-propylamino]]propyloxy}phenoxy}}äthyl}}}ester-5-methylester;
1,4-Dihydro-2,6-dimethyl-4-(m-nitrophenyl)-pyridin-3,5-dicarbonsäure-{{{3-{{p-{2-[[3-[4-(2-isopropyloxy-äthoxymethyl)-phenoxy]-2-hydroxy-propylamino]]-äthoxy}phenoxy}}propyl}}}ester-5-methylester;
1,4-Dihydro-2,6-dimethyl-4-(o-nitrophenyl)-pyridin-3,5-dicarbonsäure-{{{2-{{p-{2-[[3-[4-(2-isopropyloxy-äthoxy-methyl)phenoxy]-2-hydroxy -propylamino]]äthoxy}phenoxy}}äthyl}}}ester-5-methylester;
1,4-Dihydro-2,6-dimethyl-4-(2-chlorphenyl)-pyridin-3,5-dicarbonsäure-{{{2-{{p-{2-[[3-[4-(2-isopropyloxy-äthoxy-methyl)phenoxy]-2-hydroxy-propylamino]]äthoxy}phenoxy}}äthyl}}}ester-5-methylester;

Beispiel 15: Tabletten enthaltend 20 mg an Wirkstoff, z.B. 1,4-Dihydro-4-(m-nitrophenyl)-2,6-dimethyl-pyridin-3,5-dicarbonsäure-3-{{{2-{{p-{2-[[3-[p-(2-isopropyloxyäthoxymethyl)phenoxy]-2-hydroxy-propylamino]]äthoxy}phenoxy}}äthyl}}}ester-5-methylester, werden in folgender Zusammensetzung in üblicher Weise hergestellt:

Zusammensetzung:

| | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 16: Tabletten enthaltend 1 mg an Wirkstoff, z.B. 1,4-Diydro-4-(m-nitrophenyl)-2,6-dimethyl-pyridin-3,5-dicarbonsäure-3-{{{2-{{p-{2-[[3-[p-(2-isopropyloxyäthoxymethyl)phenoxy]-2-hydroxy-propylamino]]äthoxy}phenoxy}}äthyl}}}ester-5-methylester, werden in folgender Zusammensetzung in üblicher Weise

hergestellt:

| <u>Zusammensetzung</u>: | |
|---|---|
| Wirkstoff | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

<u>Herstellung</u>:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

<u>Beispiel 17</u>: Kapseln enthaltend 10 mg an Wirkstoff, z.B. 1,4-Dihydro-4-(m-nitrophenyl)-2,6-dimethyl-pyridin-3,5-dicarbonsäure-3-{{{2-{{p-{2-[[3-[p-(2-isopropyloxyäthoxymethyl)phenoxy]-2-hydroxy-propylamino]]äthoxy}phenoxy}}äthyl}}}ester-5-methylester, werden wie folgt auf übliche Weise hergestellt:

<u>Zusammensetzung</u>:

Wirkstoff 2500 mg
Talkum 200 mg
Kolloidale Kieselsäure 50 mg

<u>Herstellung</u>:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

<u>Beispiel 18</u>: Anstelle der in den Beispielen 15 bis 17 beschriebenen Verbindung können auch pharmazeutische Präparate als Wirkstoff, enthaltend eine andere der in den Beispielen 1 bis 14 beschriebenen Verbindungen hergestellt werden.

## Patentansprüche

1. Verbindungen der Formel

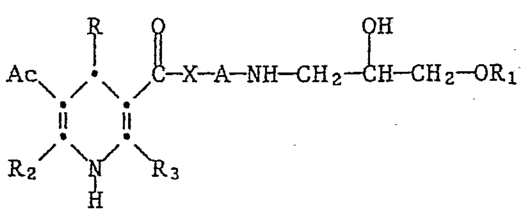

(I),

worin Ac eine Acylgruppe bedeutet, X Oxy oder gegebenenfalls substituiertes Imino bedeutet, A einen durch eine Gruppe $-X_1-Ph-X_2-$, worin mindestens einer der Reste $X_1$ und $X_2$ Oxy und ein davon verschiedener Rest $X_1$ oder $X_2$ eine direkte Bindung darstellt und Ph einen gegebenenfalls substituierten

Phenylenrest bedeutet, unterbrochenen zweiwertigen aliphatischen Kohlenwasserstoffrest darstellt, R einen gegebenenfalls substituierten Phenyl-, Pyridyl-bzw. 1-Oxidopyridyl-, (1,3-Dioxa)indanyl- oder Benzofurazanylrest bedeutet, $R_1$ einen ggf. substituierten Phenyl- oder Indolylrest bedeutet, $R_2$ einen aliphatischen Kohlenwasserstoffrest bedeutet und $R_3$ einen aliphatischen Rest, Cyano oder Amino bedeutet, und ihre Säureadditionssalze.

2. Verbindungen gemäss Anspruch 1 der Formel I, worin Ac Niederalkoxycarbonyl, Niederalkanoyl oder Niederalkansulfonyl bedeutet, X Oxy, Imino oder Niederalkylimino darstellt, A einen durch eine Gruppe der Formel $-X_1-Ph-X_2$, worin mindestens einer der Reste $X_1$ und $X_2$ Oxy und ein davon verschiedener Rest $X_1$ oder $X_2$ eine direkte Bindung darstellt und Ph einen unsubstituierten oder durch Niederalkyl, Niederalkoxy, Halogen, Trifluormethyl und/oder Nitro substituierten Phenylenrest bedeutet, unterbrochenen Alkylenrest darstellt, R einen gegebenenfalls durch Niederalkyl, Niederalkoxy, Halogenniederalkoxy, Niederalkenyloxy, Halogen, Trifluormethyl, Cyano, einen gegebenenfalls durch Niederalkyl, Niederalkoxy, Halogen, Trifluormethyl und/oder Nitro substituierten Benzylthio bzw. Benzyloxyrest und/oder Nitro substituierten Phenylrest, einen gegebenenfalls durch Niederalkyl, Niederalkoxy, gegebenenfalls S-oxidiertes Niederalkylthio und/oder Halogen substituierten, gegebenenfalls 1-oxidierten Pyridylrest oder einen (1,3-Dioxa)indanyl- oder Benzofurazanylrest bedeutet, $R_1$ einen gegebenenfalls durch Niederalkyl, Niederalkoxyniederalkyl, Niederalkoxyniederalkoxyniederalkyl, Cycloalkylniederalkoxyniederalkyl, Carbamylniederalkyl, Niederalkanoylaminoniederalkyl, Niederalkoxy, Niederalkenyloxy, Niederalkinyloxy, Niederalkoxyniederalkoxy, Carbamylniederalkoxy, Niederalkanoylaminoniederalkoxy, und/oder Cyano substituierten Phenylrest oder einen gegebenenfalls durch Niederalkyl substituierten Indolylrest bedeutet, $R_2$ für Niederalkyl steht und $R_3$ Niederalkyl, Hydroxyniederalkyl, Cyano oder Amino bedeutet, und ihre Säureadditionssalze.

3. Verbindungen gemäss Anspruch 1 der Formel I, worin Ac $C_1$-$C_7$-Alkanoyl, $C_1$-$C_7$-Alkoxycarbonyl oder $C_1$-$C_4$-Alkansulfonyl bedeutet, X für Oxy, Imino oder $C_1$-$C_4$-Alkylimino steht, A einen im Phenylenteil gegebenenfalls durch $C_1$-$C_4$-Alkyl, $C_1$-$C_4$-Alkoxy, Halogen, Trifluormethyl und/oder Nitro substituierten 8- bis und mit 14-gliedrigen $C_1$-$C_4$-Alkylenphenylenoxy-$C_2$-$C_4$-alkylenrest bzw. $C_2$-$C_4$-Alkylenoxyphenylen-$C_1$-$C_4$-alkylenrest oder 10- bis und mit 14-gliedrigen $C_1$-$C_4$-Alkylenoxyphenylenoxy-$C_1$-$C_4$-alkylenrest bedeutet, R einen gegebenenfalls durch $C_1$-$C_4$-Alkyl, $C_1$-$C_4$-Alkoxy, Mono- bis und mit Tetrahalogen-$C_1$-$C_4$-alkoxy, $C_2$-$C_4$-Alkenyloxy, einen gegebenenfalls durch $C_1$-$C_4$-Alkyl, $C_1$-$C_4$-Alkoxy, Halogen, Trifluormethyl und/oder Nitro substituiertes Benzylthio- bzw. Benzyloxyrest, Halogen, Trifluormethyl, Cyano und/oder Nitro substituierten Phenylrest, einen gegebenenfalls durch $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl und/oder Halogen substituierten Pyridyl- oder 1-Oxidopyridyl-, vorzugsweise 3-Pyridyl- oder 3-(1-Oxido)pyridylrest oder einen 4-(1,3-Dioxa)indanyl- oder 8-Benzfurazanylrest darstellt, $R_1$ einen gegebenenfalls durch $C_1$-$C_4$-Alkyl, $C_1$-$C_4$-Alkoxy-$C_2$-$C_4$-alkyl, $C_1$-$C_4$-Alkoxy-$C_2$-$C_4$-alkoxy-$C_1$-$C_4$-alkyl, 3-bis 5-gliedriges Cycloalkyl-$C_1$-$C_4$-alkoxy-$C_2$-$C_4$-alkyl, Carbamyl-$C_1$-$C_4$-alkyl, $C_2$-$C_7$-Alkanoylamino-$C_1$-$C_4$-alkyl, $C_1$-$C_4$-Alkoxy, $C_2$-$C_4$-Alkenyloxy, $C_2$-$C_4$-Alkinyloxy, ω-$C_1$-$C_4$-Alkoxy-$C_2$-$C_4$-alkoxy, Carbamyl-$C_1$-$C_4$-alkoxy, ω-$C_2$-$C_7$-Alkanoylamino-$C_2$-$C_4$-alkoxy und/oder Cyano substituierten Phenylrest oder gegebenenfalls durch $C_1$-$C_4$-Alkyl substituierten Indolylrest bedeutet, $R_2$ $C_1$-$C_4$-Alkyl darstellt und $R_3$ $C_1$-$C_4$-Alkyl, Hydroxy-$C_1$-$C_4$-alkyl, Cyano oder Amino bedeutet, und ihre Säureadditionssalze.

4. Verbindungen gemäss Anspruch 1 der Formel I, worin Ac $C_1$-$C_4$-Alkoxycarbonyl, wie Methoxycarbonyl, bedeutet, X für Oxy steht, -X-A- einen Rest der Formeln -X-alk$_1$-O-Ph-alk$_2$- oder -X-alk$_1$-O-Ph--O-alk$_3$-, worin alk$_1$ und alk$_2$ gleiche oder verschiedene $C_2$-$C_4$-Alkylenreste bedeuten, wobei alk$_1$ z.B. für Aethylen, 1,2-Propylen oder 1,2-(2-Methyl)propylen steht, und alk$_2$ $C_2$-$C_4$-Alkylen, z.B. Aethylen oder 1,3-Propylen, oder $C_1$-$C_4$-Alkyliden, z.B. Methylen oder Aethyliden, bedeutet, wie 2-(p-Aethylenphenoxy)äthoxy, 2-(p-Aethylenoxyphenyloxy)äthoxy, 2-(p-Aethylenphenoxy)äthylen, 2-[p-(2-Propylenoxy)phenoxy]äthoxy oder 2-{p-[2-(2-Methylpropylenoxy)phenoxy}äthoxy, R für unsubstituiertes oder vorzugsweise in 2- und/oder 3-Stellung durch Halogen der Atomnummer bis und mit 35, wie Chlor, mono- oder disubstituiertes oder durch Trifluormethyl, Nitro oder Cyano monosubstituiertes Phenyl bedeutet, $R_1$ gegebenenfalls durch $C_1$-$C_4$-Alkoxy-$C_2$-$C_4$-alkoxy, wie 2-Methoxyäthoxy, $C_1$-$C_4$-Alkoxy-$C_1$-$C_4$-alkyl, wie 2-Methoxyäthyl, $C_1$-$C_4$-Alkoxy-$C_2$-$C_4$-alkoxy-$C_1$-$C_4$-alkyl, wie 2-Isopropyloxyäthoxymethyl, 3- bis 5-gliedriges Cycloalkyl-$C_1$-$C_4$-alkoxy-$C_2$-$C_4$-alkyl, wie 2-Cyclopropylmethoxyäthyl, Carbamyl-$C_1$-$C_4$-alkyl, wie Carbamylmethyl oder Cyano monosubstituierten Phenyl bedeutet und $R_2$ und $R_3$ $C_1$-$C_4$-Alkyl, wie Methyl, bedeuten, und ihre Säureadditionssalze.

5. Verbindungen gemäss Anspruch 1-4 der Formel I, worin $R_1$ einen durch Halogen und/oder Niederalkanoyl substituierten Phenylrest, gegebenenfalls 1-oxidierten Pyridylrest oder einen (1,3-Dioxa)indanyl- oder Benzofurazanylrest bedeutet.

6. Verbindungen gemäss Anspruch 1-4 der Formel I, worin $R_1$ Phenyl, 2-Methyl, 4-(2-Methoxyäthyl)-, 4-(2-Isopropyloxy-äthoxymethyl)-, 4-(2-Methoxyäthoxy)-4-(2-Isopropyloxy-äthoxy)-, 4-(2-Cycloproylmethoxy-äthyl)-, 4-Carbamoylmethyl-, 2-Methoxy-, 2-Alkyloxy-, 2-Cyano-phenyl, 4-Indolyl oder 2-Methyl-4-indolyl bedeutet, und ihre Säureadditionssalze.

7. Verbindungen gemäss Anspruch 1 der Formel I, worin Ac $C_1$-$C_4$-Alkoxy-carbonyl, wie Methoxycarbonyl bedeutet, -X-A- für eine Gruppe der Formel -X-alk$_1$-O-Ph-alk$_3$- oder X-alk$_1$-O-Ph-O-alk$_3$ steht, wobei X Oxy bedeutet, alk$_1$ und alk$_3$ unabhängig voneinander $C_2$-$C_4$-Alkylen, wie Ethylen, 1,2-, 1,3-Propylen oder 1,2-(2-Methyl)-propylen, bedeuten und Ph 1,2-, 1,3- oder 1,4-Phenylen bedeutet, R für

in 2-und/oder 3-Stellung durch Halogen der Atomnummer bis und mit 35, wie Chlor, mono- oder disubstituierten, durch $C_1$-$C_4$-Alkoxy, wie Methoxy, insbesondere in Position 3, oder durch Nitro, insbesondere in Position 2 oder 3, monosubstituiertes Phenyl bedeutet, $R_1$ durch 2-Methoxyäthyl, 2-Isopropyloxy-äthoxymethyl, 2-Cyclopropylmethoxyäthyl, 2-Methoxyäthoxy oder 2-Isopropyloxy-äthoxy, insbesondere jeweils in 4-Stellung, monosubstituiertes oder durch Cyano, insbesondere in 2-Stellung, monosubstituiertes Phenyl bedeutet, und $R_2$ und $R_3$ jeweils $C_1$-$C_4$-Alkyl, wie Methyl, bedeuten, und ihre Säureadditionssalze.

8. Verbindungen gemäss Anspruch 1 der Formel I, worin Ac $C_1$-$C_4$-Alkoxy-carbonyl, wie Methoxycarbonyl, bedeutet, -X-A- für eine Gruppe der Formel -X-$alk_1$-O-Ph-$alk_3$- oder -X-$alk_1$-O-Ph-$alk_3$ steht, wobei X Oxy ist, $alk_1$ und $alk_3$ unabhängig voneinander $C_2$-$C_4$-Alkylen, wie Ethylen, 1,2-, 1,3-Propylen oder 1,2-(2-Methyl)-propylen, bedeuten und Ph 1,4-, ferner 1,2- und 1,3-Phenylen bedeutet, R für 3-Nitro- oder 3-Methoxyphenyl steht, $R_1$ 4-(2-Methoxyäthyl)-, 4-(2-Isopropyloxy-äthoxymethyl)-, 4-(2-Cyclopropylmethoxy-äthyl)-, 4-(2-Methoxyäthoxy)-4-(2-Isopropyloxy-äthoxy)-phenyl oder 2-Cyanophenyl bedeutet und $R_2$ und $R_3$ jeweils $C_1$-$C_4$-Alkyi, wie Methyl, bedeuten, und ihre Säureadditionssalze.

9. Verbindungen gemäss Anspruch 1 der Formel I, worin Ac $C_1$-$C_3$-Alkoxy-carbonyl, wie Methoxycarbonyl, bedeutet, -X-A- für eine Gruppe der Formel -X-$C_2$-$H_4$-O-Ph-$alk_3$- oder -X-$C_2H_4$-O-Ph-$alk_3$-steht, wobei X Oxy ist, Ph 1,4- oder 1,2-Phenylen bedeutet uns $alk_3$ $C_2$-$C_4$-Alkylen, wie Ethylen oder 1,2-(2-Methyl)-propylen, bedeutet, R 3-Nitro- oder 3-Methoxyphenyl bedeutet, $R_2$ und $R_3$ $C_1$-$C_4$-Alkyl, wie Methyl, bedeuten und $R_1$ 4-(2-Methoxyäthyl)-, 4-(2-Isopropyloxyäthoxymethyl)-, 4-(2-Cyclopropylmethoxy-äthyl)-, oder 4-(2-Methoxyäthoxy)-phenyl oder $R_1$ 2-Cyanophenyl bedeutet, und ihre Säureadditionssalze.

10. Verbindungen gemäss Anspruch 1 der Formel I, worin Ac $C_1$-$C_3$-Alkoxycarbonyl, wie Methoxycarbonyl, bedeutet, -X-A- für eine Gruppe der Formel -X-$C_2H_5$-O-Ph-O-$alk_3$ steht, wobei X Oxy ist, Ph 1,4-Phenylen bedeutet und $alk_3$ Ethylen oder 1,2-(2-Methyl)-propylen bedeutet, R 3-Nitrophenyl bedeutet, $R_1$ 4-(2-Methoxyäthyl)- oder 4-(2-Isopropyloxy-äthoxymethyl)-phenyl bedeutet und $R_2$ und $R_3$ $C_1$-$C_2$-Alkyl, wie Methyl, bedeuten, und ihre Säureadditionssalze.

11. 1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]äthyl}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[3-(o-cyanophenoxy(-2-hydroxy-propylamino]-2-methyl-propyloxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[p-(2-methoxyäthoxy)phenoxy]-2-hydroxypropylamino]]2-methyl-propyloxy}phenoxy}}äthyl}}}ester-5-methylester oder ein Säureadditionssalz davon.

12. 1,4-Dihydro-2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propylamino]]äthoxy}-phenoxy}}-äthyl}}}ester-5-methylester oder ein Säureadditionssalz davon.

13. 1,4-Dihydro-2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propylamino]]-2-methyl-propoxy}-phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-methoxy-äthyl)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-methoxyäthoxy)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-cyclopropylmethoxy-äthyl)phenoxy]-2-hydroxy-propylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-isopropoxy-äthoxy)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{o-{2-[[3-[4-(2-isopropoxy-äthoxy-methyl)-phenoxy]-2-hydroxy-propylamino]]äthoxy}phenoxy}}äthyl}}}-ester-5-methylester,
1,4-Dihydro-2,6-dimethyl-4-(m-methoxyphenyl)-pyridin-3,5-dicarbonsäure-3-{{{2-{{p-{2-[[3-[4-(2-isopropoxy-äthoxymethyl)-phenoxy]-2-hydroxy-propylamino]]äthoxy}phenoxy}}-äthyl}}}ester-5-methylester oder ein Säureadditionssalz davon.

14. Verbindung gemäss einem der Ansprüche 1-13 in freier Form oder in Form eines pharmazeutisch verwendbaren Säureadditionssalzes zur Anwendung in einem Verfahren zur therapeutischen oder prophylaktischen Behandlung des menschlichen oder tierischen Körpers.

15. Verbindung gemäss einem der Ansprüche 1-14 in freier Form oder in Form eines pharmazeutisch verwendbaren Säureadditionssalzes zur Verwendung als Antihypertonikum, Antianginosum oder Antiarrhythmikum.

16. Pharmazeutische Präparate enthaltend eine Verbindung gemäss einem der Ansprüche 1-14 in freier Form oder in Form eines pharmazeutisch verwendbaren Säureadditionssalzes.

17. Verwendung von Verbindungen gemäss einem der Ansprüche 1-14 zur Herstellung von Antihypertonika, Antianginosa oder Antiarrhythmika.

18. Verfahren zur Herstellung von Verbindungen gemäss einem der Ansprüche 1-14, dadurch

gekennzeichnet, dass man

a) eine Verbindung der Formel

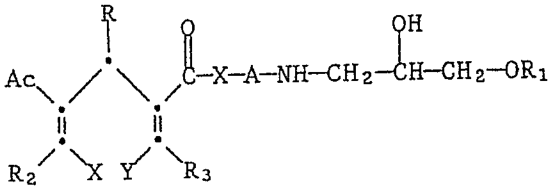

(II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

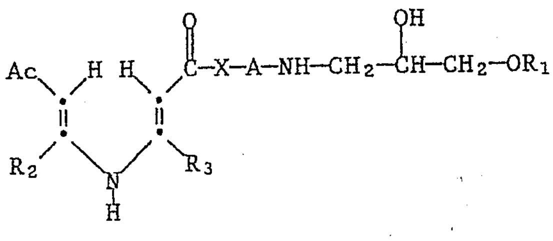

(IV),

oder einem Tautomeren davon oder einem entsprechenden Tautomerengemisch umsetzt, oder

c) in einer Verbindung der Formel

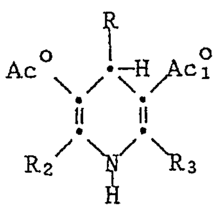

(V),

in welcher $Ac^o$ eine Gruppe -Ac oder einen in diesen überführbaren Rest und $Ac_1^o$ einen in eine Gruppe der Formel

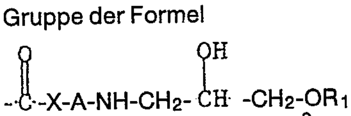 (VI) überführbaren Rest oder $Ac^o$ einen in eine Gruppe Ac überführbaren Rest und $Ac_1^o$ eine Gruppe der Formel VI bedeutet, $Ac^o$ in eine Gruppe Ac und/oder $Ac_1^o$ in eine Gruppe der Formel VI überführt, oder

d) eine Verbindung der Formel

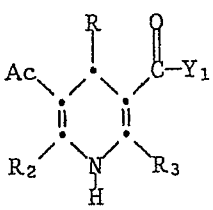

(VII)

mit einer Verbindung der Formel

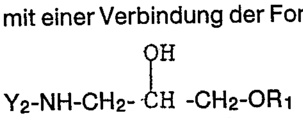 (VIII) umsetzt, worin $Y_1$ eine Gruppe Y und $Y_2$ eine Gruppe der Formel H-X-A-oder $Y_1$ eine Gruppe der Formel -X-A-Y und $Y_2$ Wasserstoff oder $Y_1$ eine Gruppe der Formel -X-H und $Y_2$ eine Gruppe der Formel Y-A-bedeutet und Y jeweils einen abspaltbaren Rest darstellt, oder

e) eine Verbindung der Formel

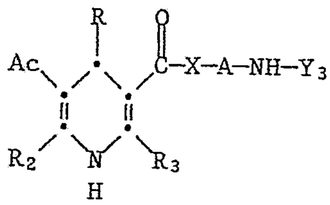 (IX)

mit einer Verbindung der Formel
$Y_4$-$OR_1$ (X)
umsetzt, worin einer der Reste $Y_3$ und $Y_4$ Wasserstoff und der andere eine Gruppe der Formel

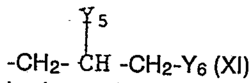 (XI)

bedeutet, in der $Y_5$ für Hydroxy und $Y_6$ für einen abspaltbaren Rest steht oder $Y_5$ und $Y_6$ gemeinsam eine Epoxygruppe darstellen, und, wenn erwünscht, eine erhaltene Verbindung der Formel I in eine andere Verbindung der Formel I unwandelt, und/oder eine erhaltene freie Verbindung der Formel I in ein Salz oder ein erhaltenes Salz in die freie Verbindung oder in ein anderes Salz überführt, umwandelt und/oder, wenn erwünscht, ein erhaltenes Isomerengemisch in die individuellen Isomere auftrennt.

19. Verfahren zur Herstellung von pharmazeutischen Präparaten gemäss Anspruch 16, dadurch gekennzeichnet, dass man eine Verbindung gemäss einem der Ansprüche 1-14 in freier Form oder in Form eines pharmazeutisch verwendbaren Säureadditionssalzes, gegebenenfalls unter Beimischung von üblichen Hilfs- und Trägerstoffen, zu pharmazeutischen Präparaten verarbeitet.

Patentansprüche für die folgenden Vertragsstaaten: AT, ES, GR

1. Verfahren zur Herstellung von Verbindungen der Formel

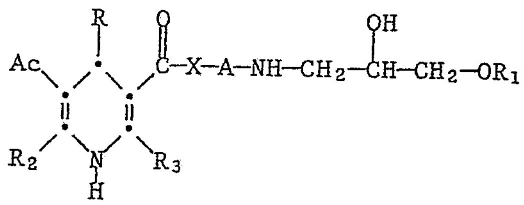 (I),

worin Ac eine Acylgruppe bedeutet, X Oxy oder gegebenenfalls substituiertes Imino bedeutet, A einen durch eine Gruppe -$X_1$-Ph-$X_2$-, worin mindestens einer der Reste $X_1$ und $X_2$ Oxy und ein davon verschiedener Rest $X_1$ oder $X_2$ eine direkte Bindung darstellt und Ph einen gegebenenfalls substituierten Phenylenrest bedeutet, unterbrochenen zweiwertigen aliphatischen Kohlenwasserstoffrest darstellt, R einen gegebenenfalls substituierten Phenyl-, Pyridyl-bzw. 1-Oxidopyridyl-, (1,3-Dioxa)indanyl- oder Benzofurazanylrest bedeutet, $R_1$ einen ggf. substituierten Phenyl- oder Indolylrest bedeutet, $R_2$ einen aliphatischen Kohlenwasserstoffrest bedeutet und $R_3$ einen aliphatischen Rest, Cyano oder Amino bedeutet, und ihrer Säureadditionssalze, dadurch gekennzeichnet, dass man

a) eine Verbindung der Formel

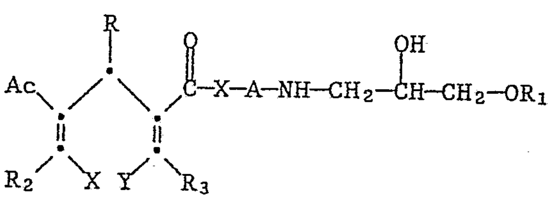 (II),

worin einer der Reste X und Y für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

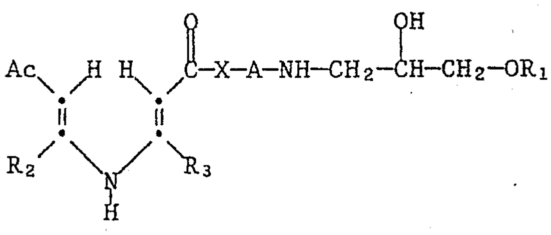 (IV),

oder einem Tautomeren davon oder einem entsprechenden Tautomerengemisch umsetzt, oder

c) in einer Verbindung der Formel

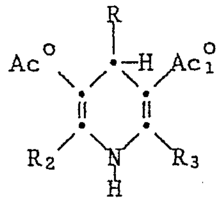 (V),

in welcher $Ac^o$ eine Gruppe -Ac oder einen in diesen überführbaren Rest und $Ac_1^o$ einen in eine Gruppe der Formel

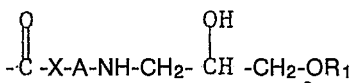 (VI) überführbaren Rest oder $Ac^o$ einen in eine Gruppe Ac überführbaren Rest und $Ac_1^o$ eine Gruppe der Formel VI bedeutet, $Ac^o$ in eine Gruppe Ac und/oder $Ac_1^o$ in eine Gruppe der Formel VI überführt, oder

d) eine Verbindung der Formel

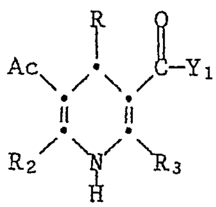 (VII)

mit einer Verbindung der Formel

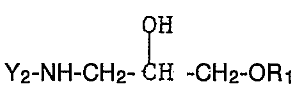 (VIII) umsetzt, worin $Y_1$ eine Gruppe Y und $Y_2$ eine Gruppe der Formel H-X-A-oder $Y_1$ eine Gruppe der Formel -X-A-Y und $Y_2$ Wasserstoff oder $Y_1$ eine Gruppe der Formel -X-H und $Y_2$ eine Gruppe der Formel Y-A-bedeutet und Y jeweils einen abspaltbaren Rest darstellt, oder

e) eine Verbindung der Formel

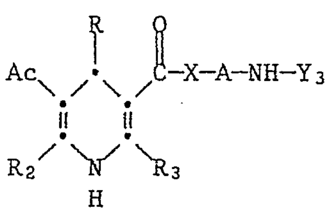 (IX)

mit einer Verbindung der Formel

$Y_4$-$OR_1$ (X)

umsetzt, worin einer der Reste $Y_3$ und $Y_4$ Wasserstoff und der andere eine Gruppe der Formel

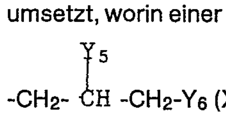 (XI)

bedeutet, in der $Y_5$ für Hydroxy und $Y_6$ für einen abspaltbaren Rest steht oder $Y_5$ und $Y_6$ gemeinsam eine Epoxygruppe darstellen, und, wenn erwünscht, eine erhaltene Verbindung der Formel I in eine andere Verbindung der Formel I umwandelt, und/oder eine erhaltene freie Verbindung der Formel I in ein Salz oder ein erhaltenes Salz in die freie Verbindung oder in ein anderes Salz überführt, umwandelt und/oder, wenn erwünscht, ein erhaltenes Isomerengemisch in die individuellen Isomere auftrennt.

2. Verfahren nach Anspruch 1 zur Herstellung von Verbindungen der Formel I, worin Ac Niederalkoxycarbonyl, Niederalkanoyl oder Niederalkansulfonyl bedeutet, X Oxy, Imino oder Niederalkylimino darstellt, A einen durch eine Gruppe der Formel $-X_1-Ph-X_2$, worin mindestens einer der Reste $X_1$ und $X_2$ Oxy und ein davon verschiedener Rest $X_1$ oder $X_2$ eine direkte Bindung darstellt und Ph einen unsubstituierten oder durch Niederalkyl, Niederalkoxy, Halogen, Trifluormethyl und/oder Nitro substituierten Phenylenrest bedeutet, unterbrochenen Alkylenrest darstellt, R einen gegebenenfalls durch Niederalkyl, Niederalkoxy, Halogenniederalkoxy, Niederalkenyloxy, Halogen, Trifluormethyl, Cyano, einen gegebenenfalls durch Niederalkyl, Niederalkoxy, Halogen, Trifluormethyl und/oder Nitro substituierten Benzylthio- bzw. Benzyloxyrest und/oder Nitro substituierten Phenylrest, einen gegebenenfalls durch Niederalkyl, Niederalkoxy, gegebenenfalls S-oxidiertes Niederalkylthio und/oder Halogen substituierten, gegebenenfalls 1-oxidierten Pyridylrest oder einen (1,3-Dioxa)indanyl- oder Benzofurazanylrest bedeutet, $R_1$ einen gegebenenfalls durch Niederalkyl, Niederalkoxyniederalkyl, Niederalkoxyniederalkoxynierderalkyl, Cycloalkylniederalkoxyniederalkyl, Carbamylniederalkyl, Niederalkanoylaminoniederalkyl, Niederalkoxy, Niederalkenyloxy, Niederalkinyloxy, Niederalkoxyniederalkoxy, Carbamylniederalkoxy, Niederalkanoylaminoniederalkoxy, und/oder Cyano substituierten Phenylrest oder einen gegebenenfalls durch Niederalkyl substituierten Indolylrest bedeutet, $R_2$ für Niederalkyl steht und $R_3$ Niederalkyl, Hydroxyniederalkyl, Cyano oder Amino bedeutet, und ihrer Säureadditionssalze.

3. Verfahren nach Anspruch 1 zur Herstellung von Verbindungen der Formel I, worin Ac $C_1$-$C_7$-Alkanoyl, $C_1$-$C_7$-Alkoxycarbonyl oder $C_1$-$C_4$-Alkansulfonyl bedeutet, X für Oxy, Imino oder $C_1$-$C_4$-Alkylimino steht, A einen im Phenylenteil gegebenenfalls durch $C_1$-$C_4$-Alkyl, $C_1$-$C_4$-Alkoxy, Halogen, Trifluormethyl und/oder Nitro substituierten 8- bis und mit 14-gliedrigen $C_1$-$C_4$-Alkylenphenylenoxy-$C_2$-$C_4$-alkylenrest bzw. $C_2$-$C_4$-Alkylenoxyphenylen-$C_1$-$C_4$-alkylenrest oder 10- bis und mit 14-gliedrigen $C_1$-$C_4$-Alkylenoxyphenylenoxy-$C_1$-$C_4$-alkylenrest bedeutet, R einen gegebenenfalls durch $C_1$-$C_4$-Alkyl, $C_1$-$C_4$-Alkoxy, Mono- bis und mit Tetrahalogen-$C_1$-$C_4$-alkoxy, $C_2$-$C_4$-Alkenyloxy, einen gegebenenfalls durch $C_1$-$C_4$-Alkyl, $C_1$-$C_4$-Alkoxy, Halogen, Trifluormethyl und/oder Nitro substituiertes Benzylthio- bzw. Benzyloxyrest, Halogen, Trifluormethyl, Cyano und/oder Nitro substituierten Phenylrest, einen gegebenenfalls durch $C_1$-$C_4$-Alkyl, $C_1$-$C_4$-Alkoxy, $C_1$-$C_4$-Alkylthio, $C_1$-$C_4$-Alkansulfinyl, $C_1$-$C_4$-Alkansulfonyl und/oder Halogen substituierten Pyridyl- oder 1-Oxidopyridyl-, vorzugsweise 3-Pyridyl- oder 3-(1-Oxido)pyridylrest oder einen 4-(1,3-Dioxa)indanyl-oder 8-Benzfurazanylrest darstellt, $R_1$ einen gegebenenfalls durch $C_1$-$C_4$-Alkyl, $C_1$-$C_4$-Alkoxy-$C_2$-$C_4$-alkyl, $C_1$-$C_4$-Alkoxy-$C_2$-$C_4$-alkoxy-$C_1$-$C_4$-alkyl, 3-bis 5-gliedriges Cycloalkyl-$C_1$-$C_4$-alkoxy-$C_2$-$C_4$-alkyl, Carbamyl-$C_1$-$C_4$-alkyl, $C_2$-$C_7$-Alkanoylamino-$C_1$-$C_4$-alkyl, $C_1$-$C_4$-Alkoxy, $C_2$-$C_4$-Alkenyloxy, $C_2$-$C_4$-Alkinyloxy, $\omega$-$C_1$-$C_4$-Alkoxy-$C_2$-$C_4$-alkoxy, Carbamyl-$C_1$-$C_4$-alkoxy, $\omega$-$C_2$-$C_7$-Alkanoylamino-$C_2$-$C_4$-alkoxy und/oder Cyano substituierten Phenylrest oder gegebenenfalls durch $C_1$-$C_4$-Alkyl substituierten Indolylrest bedeutet, $R_2$ $C_1$-$C_4$-Alkyl darstellt und $R_3$ $C_1$-$C_4$-Alkyl, Hydroxy-$C_1$-$C_4$-alkyl, Cyano oder Amino bedeutet, und ihrer Säureadditionssalze.

4. Verfahren nach Anspruch 1 zur Herstellung von Verbindungen der Formel I, worin Ac $C_1$-$C_4$-Alkoxycarbonyl, wie Methoxycarbonyl, bedeutet, X für Oxy steht, -X-A- einen Rest der Formeln $-X-alk_1-O-Ph-alk_2-$ oder $-X-alk_1-O-Ph-O-alk_3-$, worin $alk_1$ und $alk_2$ gleiche oder verschiedene $C_2$-$C_4$-Alkylenreste bedeuten, wobei $alk_1$ z.B. für Aethylen und $alk_3$ z.B. für Aethylen, 1,2-Propylen oder 1,2-(2-Methyl)propylen steht, und $alk_2$ $C_2$-$C_4$-Alkylen, z.B. Aethylen oder 1,3-Propylen, oder $C_1$-$C_4$-Alkyliden, z.B. Methylen oder Aethyliden, bedeutet, wie 2-(p-Aethylenphenoxy)äthoxy, 2-(p-Aethylenoxyphenyloxy)äthoxy, 2-(p-Aethylenphenoxy)äthylen, 2-[p-(2-Propylenoxy)phenoxy)äthoxy oder 2-{p-[2-(2-Methylpropylenoxy)phenoxy}äthoxy, R für unsubstituiertes oder vorzugsweise in 2- und/oder 3-Stellung durch Halogen der Atomnummer bis und mit 35, wie Chlor, mono- oder disubstituiertes oder durch Trifluormethyl, Nitro oder Cyano monosubstituiertes Phenyl bedeutet, $R_1$ gegebenenfalls durch $C_1$-$C_4$-Alkoxy-$C_2$-$C_4$-alkoxy, wie 2-Methoxyäthoxy, $C_1$-$C_4$-Alkoxy-$C_1$-$C_4$-alkyl, wie 2-Methoxyäthyl, $C_1$-$C_4$-Alkoxy-$C_2$-$C_4$-alkoxy-$C_1$-$C_4$-alkyl, wie 2-Isopropyloxyäthoxymethyl, 3- bis 5-gliedriges Cycloalkyl-$C_1$-$C_4$-alkoxy-$C_2$-$C_4$-alkyl, wie 2-Cyclopropylmethoxyäthyl, Carbamyl-$C_1$-$C_4$-alkyl, wie Carbamylmethyl oder Cyano monosubstituierten Phenyl bedeutet und $R_2$ und $R_3$ $C_1$-$C_4$-Alkyl, wie Methyl, bedeuten, und ihrer Säureadditionssalze.

5. Verfahren nach Anspruch 1 zur Herstellung von Verbindungen der Formel I, worin $R_1$ einen durch Halogen und/oder Niederalkanoyl substituierten Phenylrest, gegebenenfalls 1-oxidierten Pyridylrest oder einen (1,3-Dioxa)indanyl- oder Benzofurazanylrest bedeutet.

6. Verfahren nach Anspruch 1 zur Herstellung von Verbindungen der Formel I, worin $R_1$ Phenyl, 2-Methyl, 4-(2-Methoxyäthyl)-, 4-(2-Isopropyloxy-äthoxymethyl)-, 4-(2-Methoxyäthoxy)-4-(2-Isopropyloxy-äthoxy)-, 4-(2-Cycloproylmethoxy-äthyl)-, 4-Carbamoylmethyl-, 2-Methoxy-, 2-Alkyloxy-, 2-Cyanophenyl, 4-Indolyl oder 2-Methyl-4-indolyl bedeutet.

7. Verfahren nach Anspruch 1 zur Herstellung von Verbindungen der Formel I, worin Ac $C_1$-$C_4$-Alkoxy-carbonyl, wie Methoxycarbonyl bedeutet, -X-A- für eine Gruppe der Formel $-X-alk_1-O-Ph-$

$alk_3$- oder X-$alk_1$-O-Ph-O-$alk_3$ steht, wobei X Oxy bedeutet, $alk_1$ und $alk_3$ unabhängig voneinander $C_2$-$C_4$-Alkylen, wie Ethylen, 1,2-, 1,3-Propylen oder 1,2-(2-Methyl)-propylen, bedeuten und Ph 1,2-, 1,3-oder 1,4-Phenylen bedeutet, R für in 2- und/oder 3-Stellung durch Halogen der Atomnummer bis und mit 35, wie Chlor, mono- oder disubstituierten, durch $C_1$-$C_4$-Alkoxy, wie Methoxy, insbesondere in Position 3, oder durch Nitro, insbesondere in Position 2 oder 3, monosubstituiertes Phenyl bedeutet, $R_1$ durch 2-Methoxyäthyl, 2-Isopropyloxy-äthoxymethyl, 2-Cyclopropylmethoxy-äthyl, 2-Methoxyäthoxy oder 2-Isopropyloxy-äthoxy, insbesondere jeweils in 4-Stellung, monosubstituiertes oder durch Cyano, insbesondere in 2-Stellung, monosubstituiertes Phenyl bedeutet, und $R_2$ und $R_3$ jeweils $C_1$-$C_4$-Alkyl, wie Methyl, bedeuten, und ihrer Säureadditionssalze.

8. Verfahren nach Anspruch 1 zur Herstellung von Verbindungen der Formel I, worin Ac $C_1$-$C_4$-Alkoxy-carbonyl, wie Methoxycarbonyl, bedeutet, -X-A- für eine Gruppe der Formel -X-$alk_1$-O-Ph-$alk_3$- oder -X-$alk_1$-O-Ph-$alk_3$ steht, wobei X Oxy ist, $alk_1$ und $alk_3$ unabhängig voneinander $C_2$-$C_4$-Alkylen, wie Ethylen, 1,2-, 1,3-Propylen oder 1,2-(2-Methyl)-propylen, bedeuten und Ph 1,4-, ferner 1,2- und 1,3-Phenylen bedeutet, R für 3-Nitro- oder 3-Methoxyphenyl steht, $R_1$ 4-(2-Methoxyäthyl)-, 4-(2-Isopropyloxy-äthoxymethyl)-, 4-(2-Cyclopropylmethoxy-äthyl)-, 4-(2-Methoxyäthoxy)-4-(2-Isopropyloxyäthoxy)-phenyl oder 2-Cyanophenyl bedeutet und $R_2$ und $R_3$ jeweils $C_1$-$C_4$-Alkyl, wie Methyl, bedeuten, und ihrer Säureadditionssalze.

9. Verfahren nach Anspruch 1 zur Herstellung von Verbindungen der Formel I, worin Ac $C_1$-$C_3$-Alkoxy-carbonyl, wie Methoxycarbonyl, bedeutet, -X-A- für eine Gruppe der Formel -X-$C_2H_4$-O-Ph-$alk_3$- oder -X-$C_2H_4$-O-Ph-O-$alk_3$-steht, wobei X Oxy ist, Ph 1,4- oder 1,2-Phenylen bedeutet uns $alk_3$ $C_2$-$C_4$-Alkylen, wie Ethylen oder 1,2-(2-Methyl)-propylen, bedeutet, R 3-Nitro- oder 3-Methoxyphenyl bedeutet, $R_2$ und $R_3$ $C_1$-$C_2$-Alkyl, wie Methyl, bedeuten und $R_1$ 4-(2-Methoxyäthyl)-, 4-(2-Isopropyloxy-äthoxymethyl)-, 4-(2-Cyclopropylmethoxy-äthyl)-, oder 4-(2-Methoxyäthoxy)-phenyl oder $R_1$ 2-Cyanophenyl bedeutet, und ihrer Säureadditionssalze.

10. Verfahren nach Anspruch 1 zur Herstellung von Verbindungen der Formel I, worin Ac $C_1$-$C_3$-Alkoxycarbonyl, wie Methoxycarbonyl, bedeutet, -X-A- für eine Gruppe der Formel -X-$C_2H_5$-O-Ph-O-$alk_3$ steht, wobei X Oxy ist, Ph 1,4-Phenylen bedeutet und $alk_3$ Ethylen oder 1,2-(2-Methyl)-propylen bedeutet, R 3-Nitrophenyl bedeutet, $R_1$ 4-(2-Methoxyäthyl)- oder 4-(2-Isopropyloxy-äthoxymethyl)-phenyl bedeutet und $R_2$ und $R_3$ $C_1$-$C_2$-Alkyl, wie Methyl, bedeuten, und ihrer Säureadditionssalze.

11. Verfahren gemäss Anspruch 1, dadurch gekennzeichnet, dass man 1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]äthyl}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[3-(o-cyanophenoxy)-2-hydroxy-propylamino]-2-methyl-propyloxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[p-(2-methoxyäthoxy)phenoxy]-2-hydroxypropylamino]]2-methyl-propyloxy}phenoxy}}äthyl}}}ester-5-methylester oder ein Säureadditionssalz davon herstellt.

12. Verfahren gemäss Anspruch 1, dadurch gekennzeichnet, dass man 1,4-Dihydro-2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propylamino]]äthoxy}-phenoxy}}-äthyl}}}ester-5-methylester oder ein Säureadditionssalz davon herstellt.

13. Verfahren gemäss Anspruch 1, dadurch gekennzeichnet, dass man 1,4-Dihydro-2,6-dimethyl-4-(m-nitrophenyl)-pyridin-3,5-dicarbonsäure-3-{{{2-{{p-{2-[[3-[4-(2-isopropoxy-äthoxy-methyl)phenoxy]-2-hydroxy-propylamino]]-2-methyl-propoxy}-phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-methoxy-äthyl)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-methoxyäthoxy)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-cyclopropyl-methoxy-äthyl)phenoxy]-2-hydroxy-propylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{p-{2-[[3-[4-(2-isopropoxy-äthoxy)phenoxy]-2-hydroxypropylamino]]äthoxy}phenoxy}}äthyl}}}ester-5-methylester,
1,4-Dihydro-2,6-dimethyl-4-(m-nitrophenyl)-pyridin-3,5-dicarbonsäure-3-{{{2-{{o-{2-[[3-[4-(2-isopropoxy-äthoxy-methyl)-phenoxy]-2-hydroxy-propylamino]]äthoxy}phenoxy}}äthyl}}}-ester-5-methylester,
1,4-Dihydro-2,6-dimethyl-4-(m-methoxyphenyl)-pyridin-3,5-dicarbonsäure-3-{{{2-{{p-{2-[[3-[4-(2-isopropoxy-äthoxymethyl)-phenoxy]-2-hydroxy-propylamino]]äthoxy}phenoxy}}-äthyl}}}ester-5-methylester oder ein Säureadditionssalz davon herstellt.

14. Verfahren zur Herstellung pharmazeutischer Präparate, dadurch gekennzeichnet, dass man eine Verbindung gemäss einem der Ansprüche 1-13 in freier Form oder in Form eines pharmazeutisch verwendbaren Säureadditionssalzes, gegebenenfalls unter Beimischung von üblichen Hilfs- und Trägerstoffen, zu pharmazeutischen Präparaten verarbeitet.

Europäisches Patentamt

# EUROPÄISCHER RECHERCHENBERICHT

Nummer der Anmeldung

EP 87 81 0398

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 179 386 (BAYER A.G.)<br>* Ansprüche 1, 4-10 *<br>--- | 1,14-19 | C 07 D 211/90<br>C 07 D 401/04<br>C 07 D 405/04<br>C 07 D 413/04<br>C 07 D 401/12<br>C 07 D 401/14<br>C 07 D 405/14<br>C 07 D 413/14<br>A 61 K 31/44 //<br>C 07 C 125/065<br>C 07 C 79/343<br>C 07 C 93/06 |
| A | WO-A-8 602 640 (BRISTOL-MYERS CO.)<br>* Ansprüche 1, 2, 33-35, 38, 39, 57, 62-65, 69-72 *<br>--- | 1,14-19 | |
| A | EP-A-0 151 006 (YAMANOUCHI PHARMACEUTICAL CO. LTD.)<br>* Ansprüche 1, 4-6, 9 *<br>--- | 1,14-19 | |
| P,A | EP-A-0 194 750 (IMPERIAL CHEMICAL INDUSTRIES PLC)<br>* Ansprüche 1, 8-10 *<br>----- | 1,14-19 | |

RECHERCHIERTE SACHGEBIETE (Int. Cl.4)

C 07 D 211/00
C 07 D 401/00
C 07 D 405/00
C 07 D 413/00

Der vorliegende Recherchenbericht wurde für alle Patentansprüche erstellt.

| Recherchenort | Abschlußdatum der Recherche | Prüfer |
|---|---|---|
| BERLIN | 15-10-1987 | VAN AMSTERDAM L.J.P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82